# EUROPEAN PATENT APPLICATION

(11) **EP 1 729 235 A1**
(43) Date of publication of application: **06.12.2006**
(21) Application number: 06114711.2
(22) Date of filing: 30.05.2006
(51) Int. Cl.: G06F 19/00, G06F 17/30

(54) **Structured reporting report data manager**

(30) Priority: 03.06.2005 US 687605 P
(71) Applicant: Agfa Corporation, Etobicoke, NJ 07660 (US)
(72) Inventor: Kuhn, Alan, Wauwatosa, WI 53226 (US); Galbari, Scott, Waukesha, WI 53188 (US); Baumgartner, Christopher, Waukesha, WI 53188 (US)
(74) Representative: Verbrugghe, Anne Marie L.

(57) **Abstract**

Systems and methods of storing one or more report elements included in a structured report. One method includes obtaining the structured report, determining one or more report elements included in the structured report, and storing the one or more report elements in a report data database.

## Description

### FIELD OF THE INVENTION

The present invention relates to a system for processing information and creating structured reports.

### BACKGROUND OF THE INVENTION

Embodiments of the invention relate to methods and systems for reporting medical findings derived from medical information, such as information obtained from x-rays, electrocardiograms ("ECGs"), echocardiograms, CAT scans, MRIs, and the like.

Various medical specialists use a variety of imaging and patient monitoring techniques to obtain information regarding the condition of patients. Radiologists and other image specialists generally specialize in the reading and interpretation of medical images. Other specialists may be similarly skilled in interpreting ECGs and other recordings of physiological activity. In many instances, specialists read and interpret medical information for the benefit of physicians not skilled in the particular imaging or data acquisition technology. It has been common practice that specialists dictate their findings and conclusions, with written reports ultimately generated from a transcription of the dictation. Often, specialists determine the format and content of each report on a case-by-case basis.

There have been a variety of efforts to require image and patient monitoring specialists to create what are known as "structured reports." Structured reporting places requirements on the content and format of the reports produced by such specialists.

### SUMMARY OF THE INVENTION

Although the concept of structured reporting is known, and a variety of technologies have been implemented in attempts to make creating and sharing such reports easier, there are still a variety of problems associated with structured reporting systems. Accordingly, there is a need for improved methods and systems for creating and sharing structured reports.

Some embodiments of the invention provide a system for processing medical information and creating structured reports. The system may include a business logic server; a structured object repository configured to hold a plurality of structured report templates, where the structured report templates are based on a common schema; and at least one inbound message device configured to receive data from at least one source of patient data in a message-oriented protocol, to convert the data from the at least one source of patient data to a format recognized by the business logic server, and to send the converted data to the business logic server.

The business logic server may be configured to obtain at least one structured report template from the plurality of structured report templates and to create a structured report by inserting the converted data into the at least one structured report template.
The business logic server may also be configured to store the structured report in the structured object repository and to generate a completion message after creating the structured report.

In some embodiments, the system may also include a report server configured to request structured reports from the business logic server and display the reports to an end user; a service tools server having a report template editor; an outbound message device; and/or a common data store.

The at least one inbound message device may includes a device file having a poller and a link to a template and be configured to map data into a template using a code type data structure. The code type data structure may include a coding scheme designator, a code value, a code scheme version, and a code meaning

In another embodiment, the invention provides a method of creating a structured medical report. The method includes establishing a generalized structured report format; establishing a group of domain specific medical dictionaries; applying at least one of the domain specific medical dictionaries to the generalized structured report format using a processor; and inputting medical data to the processor. The method may also include creating a schema. Creating the schema may include creating a content item element, a name code element, a units code element, and an input element.

Yet another embodiment provides a data dictionary for use in a medical information system. The data dictionary may include a link to a template; a plurality of concepts associated with the templates; at least one source expression; and at least one destination expression linked to at least one of the plurality of concepts, the at least one destination expression having a plurality of arguments including a code scheme designator, a code value, a code scheme version, and a code meaning.

Additional embodiments provide a system for processing medical information and creating structured reports. The system can include a business logic server and a structured object repository configured to hold a plurality of structured report templates. The structured report templates are based on a common schema. The system also includes at least one inbound message device configured to receive data from at least one source of patient data in a message-oriented protocol, to convert the data from the at least one source of patient data to a format recognized by the business logic server, and to send the converted data to the business logic server; a report data manager; and a report data database. The business logic server can be configured to obtain at least one structured report template from the plurality of structured report templates and to create a structured report by inserting the converted data into the at least one structured report template. The report data manager can be configured to obtain the structured report, to determine one or more report elements included in the structured report, and to store the one or more report elements in the report data database. The system can also include a query engine configured to query the report data database for the one or more report elements.

Other embodiments provide a method of storing one or more report elements included in a structured medical report. The method can include obtaining the structured medical report, determining one or more report elements included in the structured medical report, and storing the one or more report elements in a report data database.

Another embodiment provides a report data manager for use in a medical information system. The report data manager can be configured to obtain a structured medical report, to determine one or more report elements included in the structured report, and to store the one or more report elements in the report data database.

Yet another embodiment provides a report data database for use in a medical information system. The report data database can be configured to store one or more report elements included in a structure report in one or more database tables. The report data database can also be configured to accept a query from a query engine.

Other features and aspects of embodiments of the invention will become apparent to those skilled in the art upon review of the following detailed description, claims, and drawings.

### STATEMENT OF THE INVENTION

1. A system for processing information and creating structured reports, the system comprising:
   - a business logic server;
   - a structured object repository configured to hold a plurality of structured report templates, the structured report templates based on a common schema;
   - at least one inbound message device configured to receive data from at least one source of data in a message-oriented protocol, to convert the data from the at least one source of data to a format recognized by the business logic server, and to send the converted data to the business logic server;
   - a report data manager; and
   a report data database, the business logic server configured to obtain at least one structured report template from the plurality of structured report templates and to create a structured report by inserting the converted data into the at least one structured report template, the report data manager configured to obtain the structured report, to determine one or more report elements included in the structured report, and to store the one or more report elements in the report data database.
2. The system of claim 1 further comprising a query engine configured to query the report data database for the one or more report elements.
3. The system of claim 1 wherein the business logic server is further configured to generate a completion message that includes the structured report and to transmit the completion message.
4. The system of claim 3 wherein the report data manager is further configured to receive the completion message.
5. The system of claim 1 wherein the report data database is configured to include at least one of a report table, a container table, and a maintenance table.
6. The system of claim 5 wherein the report data database to include at least one of a table cross-reference maintenance table, a report cross-reference maintenance table, and a last identifier maintenance table.
7. The system of claim 1 wherein the report data manager is further configured to parse the structured report and generate one or more report concept structures.
8. The system of claim 7 wherein the report data manager is further configured to obtain a current database state from the report data database.
9. The system of claim 8 wherein the report data manager is further configured to compare the report concept structures with the current database state.
10. The system of claim 9 wherein the report data manager is further configured to generate one or more table maintenance statements.
11. The system of claim 10 wherein the one or more table maintenance statements include at least one of a create table statement and an alter table statement.
12. The system of claim 11 wherein the report data manager is further configured to order the one or more table maintenance statements.
13. The system of claim 11 wherein the report data manager is further configured to execute the one or more table maintenance statements.
14. The system of claim 11 wherein the report data manager is further configured to roll back the execution of the one or more table maintenance statements.
15. The system of claim 7 wherein the report data manager is further configured to determine if the one or more report concept structures have been previously stored to the report data database.
16. The system of claim 15 wherein the discrete data manger is further configured to generate one or more table modification statements.
17. The system of claim 16 wherein the one or more table modification statements include an update statement.
18. The system of claim 16 wherein the one or more table modification statements include an insert statement.
19. The system of claim 16 wherein the report data manager is further configured to order the one or more table modification statements.
20. The system of claim 16 wherein the report data manager is further configured to execute the one or more table modification statements.
21. The system of claim 20 wherein the report data manager is further configured to roll back the execution of the one or more table modification statements.
22. A method of storing zero or more report elements included in a structured report, the method comprising:
   - obtaining a structured report;
   - determining zero or more report elements included in the structured report; and
   - storing the zero or more report elements in a report data database.
23. The method of claim 22 further comprising parsing the structured report and generating one or more report concept structures.
24. The method of claim 23 further comprising obtaining a current database state from the report data database.
25. The method of claim 24 further comprising comparing the one or more report concept structures with the current database state.
26. The method of claim 25 further comprising generating one or more table maintenance statements.
27. The method of claim 26 wherein the one or more table maintenance statements include at least one of a create table statement and an alter table statement.
28. The method of claim 26 further comprising ordering the one or more table maintenance statements.
29. The method of claim 26 further comprising executing the one or more table maintenance statements.
30. The method of claim 29 further comprising rolling back the execution of the one or more table maintenance statements.
31. The method of claim 23 further comprising determining if the one or more report concept structures have been previously stored to the report data database.
32. The method of claim 31 further comprising generating one or more table modification statements.
33. The method of claim 32 wherein the one or more table modification statements include an update statement.
34. The method of claim 32 wherein the one or more table modification statements include an insert statement.
35. The method of claim 32 further comprising ordering the one or more table modification statements.
36. The method of claim 32 further comprising executing the one or more table modification statements.
37. The method of claim 33 further comprising rolling back the execution of the one or more table modification statements.
38. A report data manager configured to receive a structured report, to determine one or more report elements included in the structured report, and to store the one or more report elements in the report data database.
39. The report data manager of claim 38 further configured to parse the structured report and generate one or more report concept structures.
40. The report data manager of claim 39 further configured to obtain a current database state from the report data database.
41. The report data manager of claim 40 further configured to compare the report concept structures with the current database state.
42. The report data manager of claim 41 further configured to generate one or more table maintenance statements.
43. The report data manager of claim 42 wherein the one or more table maintenance statements include at least one of a create table statement and an alter table statement.
44. The report data manager of claim 42 further configured to order the one or more table maintenance statements.
45. The report data manager of claim 42 further configured to execute the one or more table maintenance statements.
46. The report data manager of claim 45 further configured to roll back the execution of the one or more table maintenance statements.
47. The report data manager of claim 39 further configured to determine if the one or more report concept structures have been previously stored to the report data database.
48. The report data manager of claim 47 further configured to generate one or more table modification statements.
49. The report data manager of claim 48 wherein the one or more table modification statements include an update statement.
50. The report data manager of claim 48 wherein the one or more table modification statements include an insert statement.
51. The report data manager of claim 48 further configured to order the one or more table modification statements.
52. The report data manager of claim 48 further configured to execute the one or more table modification statements.
53. The report data manager of claim 52 further configured to roll back the execution of the one or more table modification statements.
54. A report data database configured to store one or more report elements included in a structured report in one or more database tables.
55. The report data database of claim 54 further configured to accept a query from a query engine.
56. The report data database of claim 55 further configured to return one or more report elements to the query engine.
57. The report data database of claim 54 wherein the one or more database tables includes at least one of a report table, a container table, and a maintenance table.
58. The report data database of claim 57 wherein the one or more database tables includes at least one of a table cross-reference maintenance table, a report cross-reference maintenance table, and a last identifier maintenance table.
59. The report data database of claim 54 further configured to accept a current database state request from a report data manager.
60. The report data database of claim 59 further configured to return a current database state to the report data manager.
61. The report data database of claim 54 further configured to create a database table based on a create table statement.
62. The report data database of claim 54 further configured to alter the one or more database tables based on one or more alter table statements.
63. The report data database of claim 54 further configured to determine if one or more report elements have been previously stored in the one or more database tables.
64.The report data database of claim 54 further configured to update one or more report elements previously stored in the one or more database tables based on one or more update statements.
65.The report data database of claim 54 further configured to store one or more report elements in the one or more database tables based on one or more insert statements.
66. A report data manager configured to provide data storage for report elements included in a qualifying structured report in a predictable set of tables and columns.

### BRIEF DESCRIPTION OF THE DRAWINGS

Fig. 1 is a schematic illustration of an exemplary system for creating structured reports.
Fig. 2 is a tree view of an exemplary extensible markup language schema definition.
Fig. 3 is an exemplary screen shot from a report template editor application.
Fig. 4 is an exemplary screen shot from a concept editor application.
Fig. 5 is a schematic diagram of hardware inside one of the devices shown in Fig. 1.
Fig. 6 is a schematic diagram illustrating software that may be stored in the memory illustrated in Fig. 5.
Fig. 7 is an exemplary screen shot from a mapping editor application.
Fig. 8 is a flow chart illustrating an exemplary process of creating a hierarchically structured report from flat report data.
Fig. 9 is a schematic diagram illustrating exemplary interactions and data flow between components of the system of Fig. 1 when creating a structured report.
Fig. 10 is a schematic diagram illustrating exemplary interactions and data flow between components of the system of Fig. 1 when notifying a component of the creation of a structured report.
Fig. 11 is a schematic diagram illustrating exemplary interactions and data flow between components of the system of Fig. 1 when viewing a structured report.
Fig. 12 is a schematic diagram illustrating exemplary interactions and data flow between components of the system of Fig. 1 when storing a created report to an external storage device.
Fig. 13 is a schematic diagram illustrating exemplary interactions and data flow between components of the system of Fig. 1 when fetching created reports from an external storage device.
Fig. 14 is a schematic diagram illustrating exemplary interactions and data flow between components of the system of Fig. 1 when creating a structured report and storing report elements to a report data database with a report data manager.
Fig. 15 is a schematic diagram illustrating exemplary table types of the report data database of Fig. 14.
Fig. 16 is a schematic diagram illustrating exemplary maintenance tables of the report data database of Fig. 14.
Fig. 17 is a schematic diagram illustrating an exemplary report structure processed with the report data manager of Fig. 14.
Figs. 18A and 18B are flow charts illustrating an exemplary process of processing a structured report with the report data manager of Fig. 14.

### DETAILED DESCRIPTION OF THE INVENTION

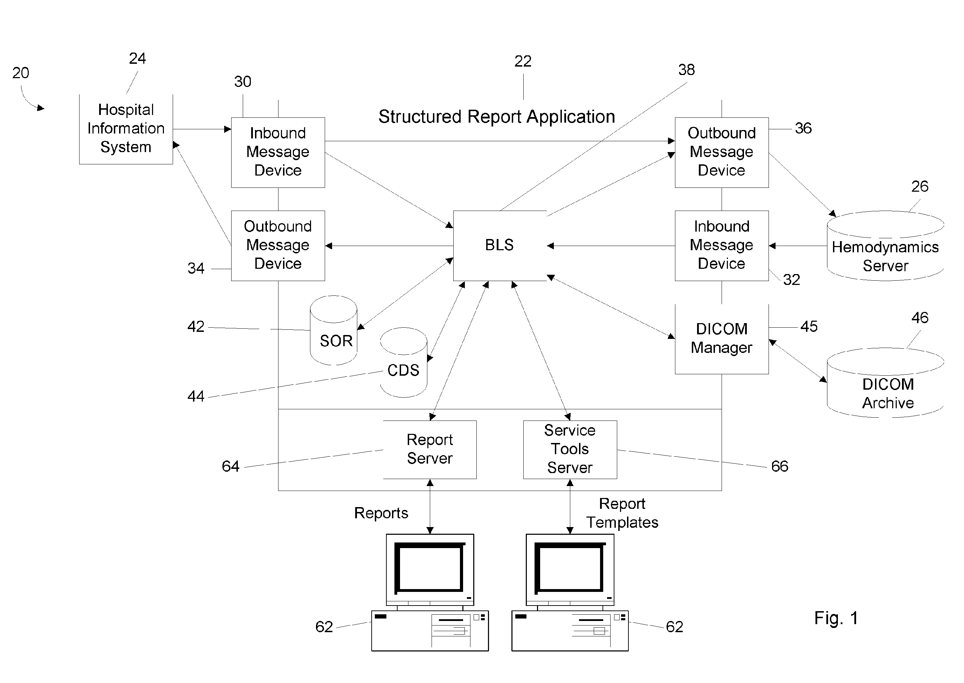

Fig. 1 illustrates an exemplary system 20 that may be used to create structured reports. The components of the system 20 are connected through the illustrated links. In reality, one or more networks or communication systems such as the Internet, the telephone system, wireless networks, satellite networks, cable TV networks, and various other private and public networks could be used in various combinations to provide the communication links desired or needed to create embodiments or implementations of the invention, as would be apparent to one of ordinary skill in the art. Thus, the invention is not limited to any specific network or combinations of networks. Data can be transferred from one party or component to another with wires, fiber optics, wireless communications, or physical media being physically carried from one party or component to another.

In Fig. 1, the system 20 represents a medical system. The medical system 20 includes a structured report application 22. The structured report application ("SR application") 22 receives input from a number of devices. In one embodiment, the SR application 22 receives data from a hospital information system 24 ("HIS") and a hemodynamics server 26. Other input and procedure devices are possible including other specialty servers providing procedure results similar to the hemodynamics server 26 such as a neurology server, a source of radiological information, or the like. Data transmitted by the HIS 24 and hemodynamics server 26 may be packaged and transmitted according to a specific protocol. For example, the devices may utilize the health level 7 ("HL7") protocol to format messages sent to the SR application 22. HL7 has a message-oriented architecture (in contrast to client-server or document architectures). In message-oriented architectures the application where an event occurs sends a message to other applications rather than servicing a request. In a medical or healthcare environment, an HL7 message may be sent by an application such as the HIS 24 or hemodynamics server 26 when a patient checks in, is transferred, or discharged; when a procedure is scheduled; when a procedure is completed; or when other events have occurred. An exemplary HL7 message that may be generated when a patient checks into a hospital or clinic is illustrated below.

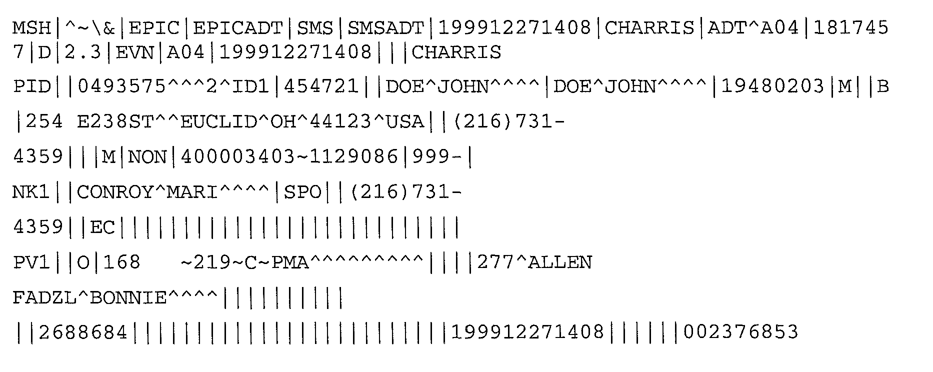

The HL7 protocol defines the type of data that may be included in a message, but does not specify or require a format for the data. Two applications or systems may generate an HL7 message regarding the transfer of a patient and both messages will contain the same data, but the format of the data in the two messages may be different. For example, one application or system may record the gender of a patient as "MALE" or "FEMALE" while another application records gender as "M" or "F."

When an application or device generates and transmits an HL7 (or other format) message, other applications or devices can listen for the messages. The SR application 22 may include one or more inbound message devices 30 and 32 configured to listen for and receive messages from devices such as the HIS 24. The inbound message devices 30 and 32 may be configured to parse and interpret the data contained within a received message to an internal format recognized and useable by the SR application 22.

The inbound message devices 30 and 32 may be configured to determine whether the data contained within the message is data that the SR application 22 should be made aware of. If the received message does not contain data needed by the SR application 22, the inbound message devices 30 and 32 may forward the message to one or more outbound message devices 34 and 36. The outbound message devices 34 and 36 may be configured to redirect the message to an application or device needing the message. For example, if a procedure for an electrocardiogram ("ECG") is scheduled on the HIS 24 and a corresponding HL7 message is generated and transmitted to the SR application 22, the SR application 22 may not require the data contained in the message, but the hemodynamics server 26 may require the data. The inbound message device 30 and 32 that receives the message may transmit the message to the hemodynamics server 26 using one of the outbound message devices 34 and 36 so that the hemodynamics server 26 may perform preparatory functions for the scheduled procedure. The inbound message devices 30 and 32 may format the received message before forwarding it to one of the outbound message devices 34 and 36. An attribute/value pairs protocol with sequenced items, such as the Mitra Common Framework ("MCF") protocol, may be used to forward the data contained within the received message from one of the inbound message devices 30 and 32 to one of the outbound message devices 34 and 36. The inbound message devices 30 and 32 and outbound message devices 34 and 36 may also communicate using other protocols such as the HL7 protocol. The inbound message devices 30 and 32 may also be configured to simply ignore the message and rely on other devices requiring the data to also be listening for the message.

If the data contained in the message received by the inbound message devices 30 and 32 is determined to be data that the SR application 22 can use, the inbound message devices 30 and 32 may send the data in a message to a business logic server ("BLS") 38. The inbound message devices 30 and 32 may also send a "report generation request" message or other instruction message to the BLS 38 in order to specify what should be done with the data. The inbound message devices 30 and 32 may communicate with the BLS 38 using the MCF protocol or other proprietary message protocols. In some embodiments, the inbound message devices 30 and 32 process the data in the message before sending the data or a message to the BLS 38, as will be described in detail below.

Upon receiving the data and/or message from the inbound message devices 30 and 32, the BLS 38 may obtain an instance of a structured report template from a structured object repository ("SOR") 42. In some embodiments, the BLS 38 may query or send a request message to the SOR 42 for the structured report template using the MCF protocol, although other messaging protocols are possible depending on the configuration of the BLS 38 and SOR 42. The BLS 38 may determine the structured report template required based on the data received. Alternatively, the message received from one of the inbound message devices 30 and 32 may include a structured report template identifier that specifies the particular template to use. The SOR 42 may contain templates for x-ray reports, ECG reports, catheterization reports, echocardiogram reports, CAT scan reports, MRI reports, and the like. Structured report templates may also be stored in other storage devices. The BLS 38 may also internally store the templates.

The structured report template specifies the data to be presented and the structure of the data in the generated report. After the BLS 38 receives the instance of the structured report template from the SOR 42, the BLS 38 inserts the received data into the template as specified to generate a structured report. The BLS 38 may require additional data other than that sent from the inbound message devices 30 and 32, and may query a common data store ("CDS") 44 in order to obtain additional data not sent from the inbound message device when a report is requested. The BLS 38 may query or send a request message to the CDS 44 using the MCF protocol or other messaging protocol. The CDS 44 may contain general patient, procedure order, or procedure study data and other demographic data.

The CDS 44 may also contain past procedure results that may be incorporated in the currently requested report. The BLS 38 may also perform calculations and/or modifications on the received data as specified in the report template. The BLS 38 may also obtain data from previously generated reports stored in the SOR 42 or other storage locations or devices.

In some embodiments, after the BLS 38 has generated the structured report, the BLS 38 saves the structured report to the SOR 42. The SOR 42 may only temporarily store the completed structured report. The BLS 38 may output the structured report to a persistent database or storage device. The BLS 38 may also convert the structured report to a particular format, such as a digital imaging and communications in medicine ("DICOM") format, before storing the generated report to a storage location external to the SR application 22. The BLS 38 may be configured to convert the structured report to a number of vendor specific formats, allowing the structured reports to be circulated and used across a number of systems, networks, and platforms. As illustrated in Fig. 1, the BLS 38 may interact with a DICOM manager 45 in order to convert and store structured reports in a DICOM report format. The DICOM manager 45 may be used by the BLS 38 to obtain conversion codes and/or formats for converting structured reports to DICOM-formatted reports. The BLS 38 and DICOM manager 45 may communicate using the MCF protocol or an alternative messaging protocol. The DICOM manager 45 may also perform the translation from a BLS-generated structured report to a DICOM-formatted report. The DICOM manager 45 may also be used to manage a DICOM data storage or archive 46. The DICOM manager 45 may store and retrieve DICOM-formatted structured reports to and from the DICOM archive 46. The DICOM manager 45 may transmit messages and data to the DICOM archive 46 using a DICOM specific protocol or other messaging protocol like HL7 or MCF.

In some embodiments, upon completing a structured report, the BLS 38 may generate a "completion" message indicating the creation of a new report. Other components and devices in the SR application 22 may listen for the "completion" message and may export the stored structured report to a secondary location after the BLS 38 stores the generated report in the SOR 42. Components or devices receiving the "completion" message may also generate a message to be sent to devices or components external to the SR application 22. For example, one of the outbound message devices 34 and 36 may listen for a "completion message" from the BLS 38 and may generate a message for the HIS 24 or other external component indicating the existence of the new report. The outbound message devices 34 and 36 may also include the location and/or name of the report in the message. The outbound message devices 34 and 36 may also be configured to obtain the new report and directly send the report data in a message to another component or device.

After the BLS 38 has generated and stored the structured report, a user may use a workstation 62 to view the generated report. The workstation 62 may interact with a report or web server 64 in order to access and view the generated report. The user queries the report server 64 using the workstation 62 for a specific report, and the report server 64 requests the specified report from the BLS 38. The report server 64 may receive the request from the workstation 62 and may forward the request, or create and transmit a new, specifically formatted request, to the BLS 38. The BLS 38 obtains the requested report from the SOR 42, DICOM archive 46, or other storage device, and returns the report to the report server 64. The report server 64 displays the returned report to the user on the workstation 62. The workstation 62 may transmit queries, requests, or forms to the report server 64 using hypertext transport protocol ("HTTP") or similar protocols, such as transmission control protocol/Internet protocol ("TCP/IP"). The report server 64 may also communicate with the BLS 38 using HTTP, MCF, HL7, or other transmitting protocols. The workstation 62 may also directly communicate with the BLS 38.

To simplify the data generated and stored for a report, a stylesheet, such as an extensible stylesheet language ("XSLT") stylesheet, may be created to provide specific display formatting for a report. The stylesheet describes how the report should be displayed. The report server 64, BLS 38, or workstation 62 may apply a stylesheet to a report to add presentation data to the report. The presentation data may include adding graphical headers specifying the hospital or system name, trademark, or logo; labeling sections of the report; formatting information, such as the size and style of the font used in the report, or the like. The BLS 38 may retrieve the stylesheet from the SOR 42, DICOM archive 46, or other storage device when it retrieves the report. The report server 64 may also retrieve the stylesheet from an internal or remote storage area. The stylesheet may also be defined and stored on the workstation 62.

In some embodiments, the report server 64 may be configured to allow a user to modify a report displayed on the workstation 62. The user may modify data, add comments, link images, or the like, using the workstation 62 and input peripherals such as a keyboard or cursor control device (not shown). The report server 64 may also be configured to display reports in multiple formats depending on the origin of the display request. For example, a user may use a browser application to request a report over an Internet, local area network (LAN), or other network connection. The report server 64 may generate a portable document format ("PDF") or other common format of the report returned by the BLS 38 so that a specific display application is not required to have specific knowledge of how to render the report. In some embodiments, editing the displayed report, however, may only be available when using a specific report viewing application.

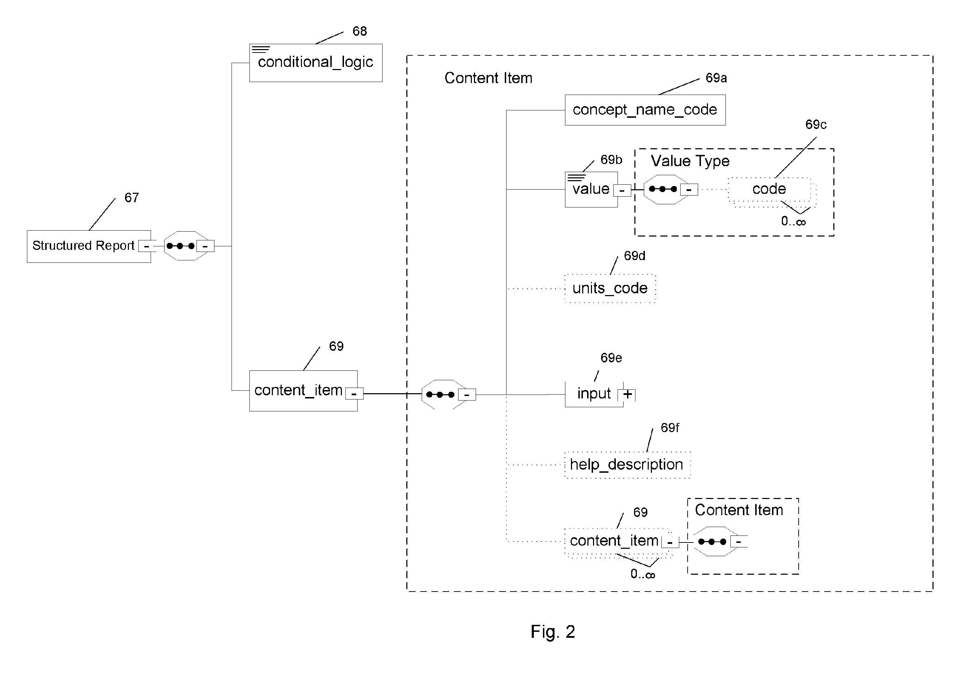

In certain embodiments, each template stored in the SOR 42 is based on a common schema. In some embodiments, the schema may be an extensible markup language ("XML") schema definition ("XSD") that specifies the data elements recognized by the BLS 38 and the corresponding formats and/or codes. The XSD defines the possible elements that may be contained in a structured report and provides structure or organization for the data. Fig. 2 illustrates a tree diagram for an exemplary XSD.

As illustrated in Fig. 2, the format of the XSD includes a StructuredReport element 67 as the root element. The StructuredReport element 67 contains all of the elements of the report. Under the StructuredReport element 67 is a conditional_logic element 68 that provides formatting of the report for described circumstances. For example, the conditional_logic element 68 may contain logic indicating that blood pressure values greater than 150 should be highlighted in red when displayed on the report. Other formatting provided through the conditional_logic element 68 may include adding headers or page numbers to every page, specifying the number of lines to appear per page, setting a font size, or the like.

Data inserted into the report ("concepts") are contained in content_item elements 69. A content_item element 69 consists of a number of attributes or sub-elements. Exemplary sub-elements are shown enclosed in the dashed-line box labeled "Content Item."

A content_item element 69 may have a concept_name_code element 69a. A concept_name_code element 69a represents a medical term or concept. For example, a concept may be an anatomical reference such as "Left Ventricle" or a diagnosis such as "Myocardial Infarction." In some embodiments, each concept_name_code element 69a has a code type data structure. A code type data structure may include four attributes: a coding scheme designator, a code value, a coding scheme version, and a code meaning. The coding scheme designator indicates the scheme that defines the concept. An exemplary scheme referenced by a coding scheme designator may include a DICOM scheme. The coding scheme version defines the version of the coding scheme indicated by the coding scheme designator that defines the concept. The code value defines a unique code for the concept that is recognized by the scheme, and the code meaning provides a text string indicating the actual concept or term. An exemplary concept_name_code element 69a may have the following structure.
<concept_name_code meaning="Patient Name" scheme="DICOM" value="0010,0020" version="3.0">

Each content_item element 69 may also comprise a value element 69b corresponding to the concept_name_code element 69a. The value element 69b represents the actual data or measurement described by the concept_name_code element 69a. For example, a content_item element 69 may have a concept_name_code element 69a of "Patient Name" and a value element 69b of "John Doe." The concept_name_code element 69a describes what the value element 69b represents.

Each value element 69b may be one of two types. A value element 69b may have a data type value and specify actual data to be recorded in the report. The data type may be a character string such as the actual patient name or a numerical value such as a measurement value. Alternatively, a value element 69b may have a valuetype type data structure, where the valuetype type data structure includes one or more code sub-elements 69c. Each code sub-element 69c may be a code type data structure as described for the concept_name_code element 69a. A value element 69b may have one or more code sub-elements and the concept defined by the concept_name_code element 69a can be represented by a constant or coded value. For example, when recording a patient's gender, instead of placing a character string of "M" or "Male," a code type data structure can be used to specify the gender. Using a coded value having a code type data structure creates uniformity across reports and allows data to be recognized and shared across reports and systems utilizing the reports. A value element 69b may have multiple code sub-elements and the concept can be defined to represent a number of constant or coded values.

Each content_item element 69 may also include other attributes including a units_code element 69d. The units_code element 69d may be used to designate measurement units for the content_item element 69. If, for example, the code element 69c is set to a number type, the units_code element 69d may specify the units for the number value such as centimeters, millimeters, seconds, beats per minute, or the like. As noted by the dotted-lines shown in Fig. 2, in some embodiments a content_item element 69 may not require a units_code element 69d. A content_time element 69 representing a patient's name, for example, may not require measurement units.

Each content_item element 69 may further include an input element 69e. The input element 69e designates restrictions or characteristics of the data input into the value element 69b. The input element 69e may specify the type of the input data such as date, date and time, text, numerical, or the like. The input element 69e may also specify whether the data for the concept_item element is required to generate the report. The input element 69e may also indicate whether the input data should be displayed on the report or hidden. Calculations to be performed on the input data may also be specified in the input element 69e. Each input element 69e may also provide validation requirements for content_item elements 69 by specifying a maximum, minimum, or data range. When generating a report, the BLS 38 can use data ranges specified in the template to verify content_item elements 69. Invalid content_item elements 69 may be excluded from the report or marked as invalid on the report. The BLS 38 may also be configured to convert the invalid content_item elements 69 into valid elements 69 before placing the data into a report. The BLS 38 may also log a message indicating the details of the error for later reference.

A content_item element 69 may also have a help_description element 69f which specifies instructions or hints for the content_item element 69. The help_description element 69f may display descriptive text to a user when the user views or edits the content_item element 69. The descriptive text may describe the content_item such as how the measurement is taken, how the measurement is being displayed, normal data ranges for the measurement, how to edit the measurement, or the like. In some embodiments, a content_item element 69 does not require a help_description element 69f, as indicated by the dotted lines surrounding the help_description element 69f.

A content_item element 69 can be a "container" or a multicontainer element that contains nested content_item elements 69. A multicontainer element may represent a tree of nodes or content_item elements 69 that may, in some embodiments, be repeatable. Nested content_item elements 69 contained in the mulitcontainer tree may indicate relationships between data items. Each multicontainer element may contain multiple child content_item elements 69 and may be repeated multiple times. For example, a multicontainer element holding vital signs of a patient may contain a content_item element 69 representing a heart rate and another content_item element 69 representing a blood pressure. Also, if multiple sets of vital signs are obtained for a patient, for example, a study of vital signs over a three hour procedure, multiple vital signs multicontainers may be present in the structured report generated. In some embodiments, there is no limit to the number of multicontainer elements specified in a report nor is there a limit to the number of content_item elements 69 contained within the multicontainer elements.

Specific content_item elements 69 may also be added multiple times to a template. For example, the patient's name or identification number may appear multiple times on a report and may appear in various formats. Also, a measurement may consist of many content_item elements 69 if multiple measurements of the same type are taken during a procedure. For example, blood pressure values over a week's time may be listed on a report. The measurements may be treated as a group of content_item elements 69, or as a single content_item, so that they can be properly mapped and placed in a report.

The XSD represents a common thread that components of the SR application 22 use to communicate and understand data and reports generated for various components of the system 20. Since report templates are based on the common XSD, not only can, for example, two x-ray structured reports be understood, compared, combined, and analyzed, but an x-ray structured report and MRI structured report can be understood, compared, and analyzed by any device or component that knows the XSD.

In some embodiments, templates may be created for specific reports through a report template generator or editor application provided by a service tools server 66. The service tools server 66 may also store various administrative tools used to configure, monitor, or analyze the SR application 22 or the components contained therein. The service tools server 66 may also contain authentication applications to validate users before they are allowed to modify components of the SR application 22.

The structured report templates may be created ahead of time or may be created or dynamically modified as needed to customize a structured report. In some embodiments, modifying a template may create a new version of the template rather than overriding the previous format of the template. To generate a report template a user may use a workstation 62 to execute the report template editor. The report template editor application may be downloaded to the workstation 62 from the service tools server 66 and executed by a processor of the workstation 62. The workstation 62 may also be configured to submit forms or requests and receive output from the service tools server 66, where the template editor application is executed. In some embodiments, the report template editor application may also be stored locally and executed on the workstation 62.

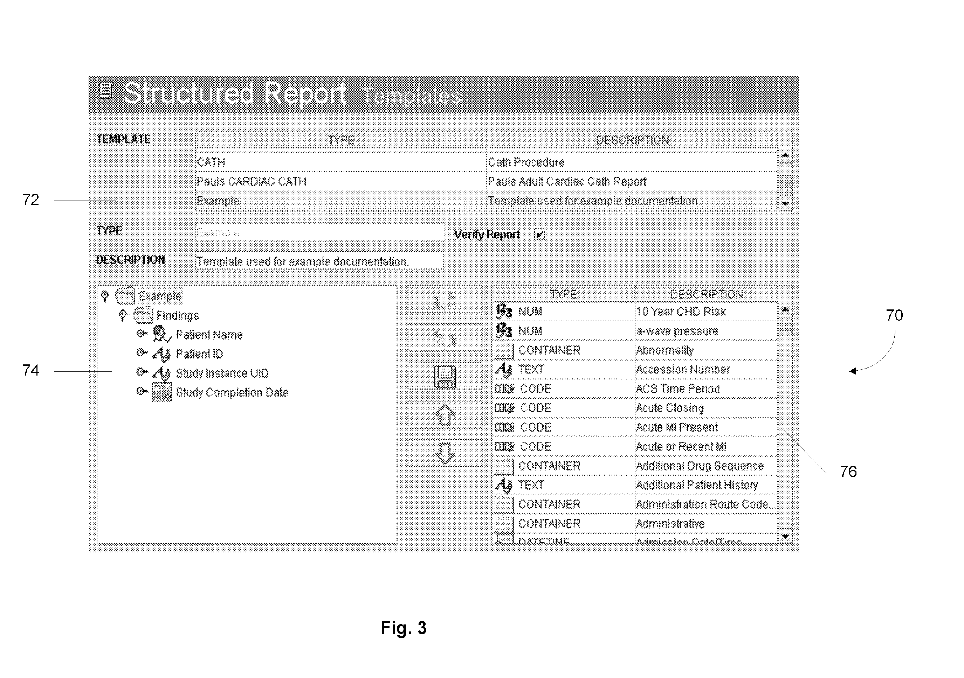

Fig. 3 illustrates an exemplary screen shot 70 from the report template editor application. The exemplary screen 70 consists of three areas. The top of the screen 70 displays a template list 72 that catalogs all of the existing templates. In some embodiments, to select a specific template from the template list 72 (to, for example, view in closer detail or to modify the template), the user may double click the template in the template list 72. The template list 72 may also include an empty template that a user may use to generate a new template. The user may also click on an icon or select an action from a drop down menu to create a new template.

The bottom left of the screen 70 contains a template descriptor 74 that displays the currently selected template. The details and components of the selected template are displayed in a tree control. Data items of the template that contain child or nested data items can be expanded in order to view their children or dependents.

The bottom right of the screen 70 includes a concept list 76 that lists the concepts recognized by the template editor application. In the example shown, a concept is a medical term. For example, a concept may be an anatomical reference such as "Left Ventricle" or a diagnosis such as "Myocardial Infarction." Each concept may have a unique code that distinctively identifies the concept. Some concepts may only be applicable to particular report types and may not be displayed in the concept list 76 if the type of the currently selected template is unable to support the concept. For example, the concept for "Left Ventricle" may not be available for a CAT scan report of a patient's cranium.

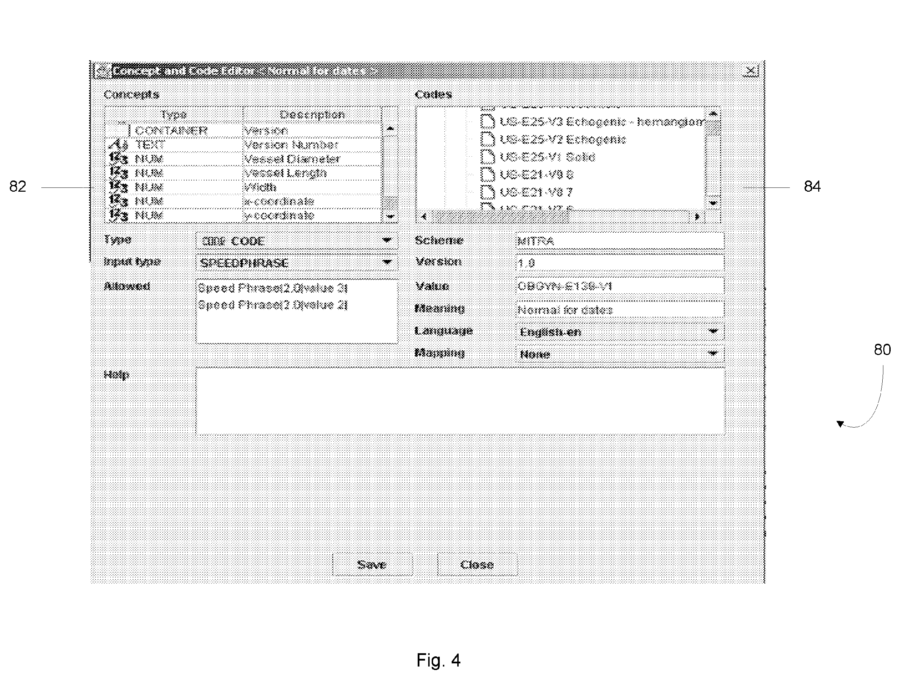

In some embodiments, the user may also add new concepts to the concept list 76 or modify existing concepts by using a concept editor application. The concept editor application may be a configuration tool provided by the service tools server 66 and may be part of the template editor application. Fig. 4 illustrates an exemplary screen shot 80 from the concept editor application. The screen shot 80 includes a concept list 82 on the left side of the screen 80 and a codes list 84 on the right. The concept editor application may be configured to restrict the removal of concepts due to legacy templates. When creating a new concept, a user may specify parameters of the concept including the type of the concept, the input type, and allowed values for the concept. In the embodiment shown, each concept is described by a code type. Available codes may be displayed and chosen from the codes list 84. In some embodiments, codes can also be created and modified using the concept editor application. A separate application may also be used to edit and add codes.

In order to create a structured report template using the template editor application, a user selects concepts from the concept list 76 and adds the concepts to the template descriptor 74. For example, if a user wishes to create a report that contains a patient name field, a patient ID field, a study instance UID field, and a study completion data field, the user selects those fields from the concept list 76 on the template editor screen 70 and moves the fields to the template descriptor area 74. The user may move the fields by double-clicking on the field in the concept list 76, by clicking and dragging the field from the concept list 76 to the template descriptor area 74, or by using another mechanism such as an icon, key combination, or menu selection. Selecting a field from the concept list 76 may not remove the field from the concept list 76, since a field may be allowed to appear on a structured report multiple times.

In some embodiments, once the user has selected all of the desired fields, the template editor application generates the following exemplary template that is transmitted to the BLS 38 for storage in the SOR 42. A structured report template can include an extensible markup language ("XML") file that uses XML to describe the structure of a structured report. 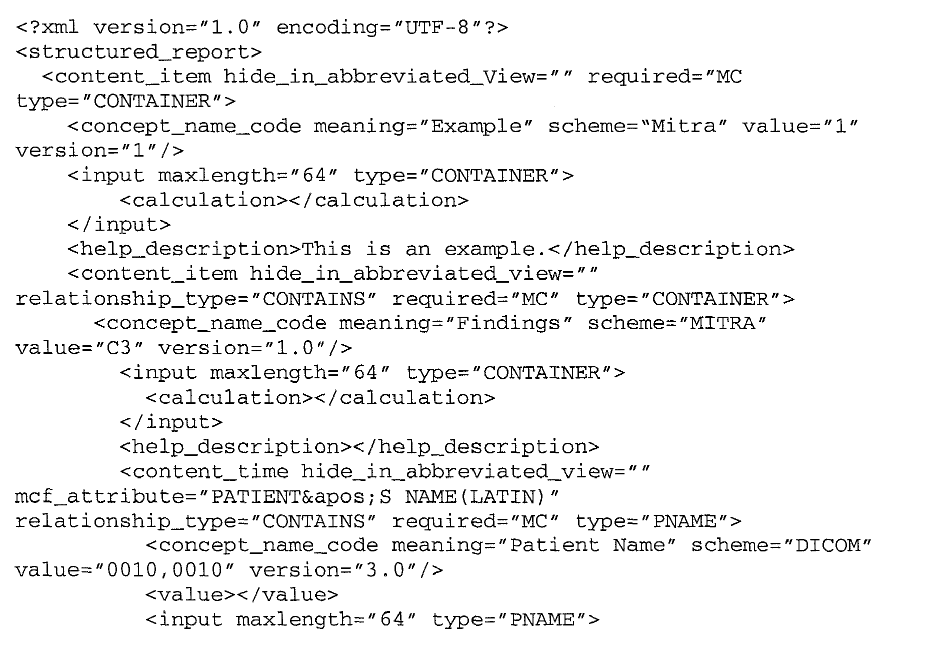 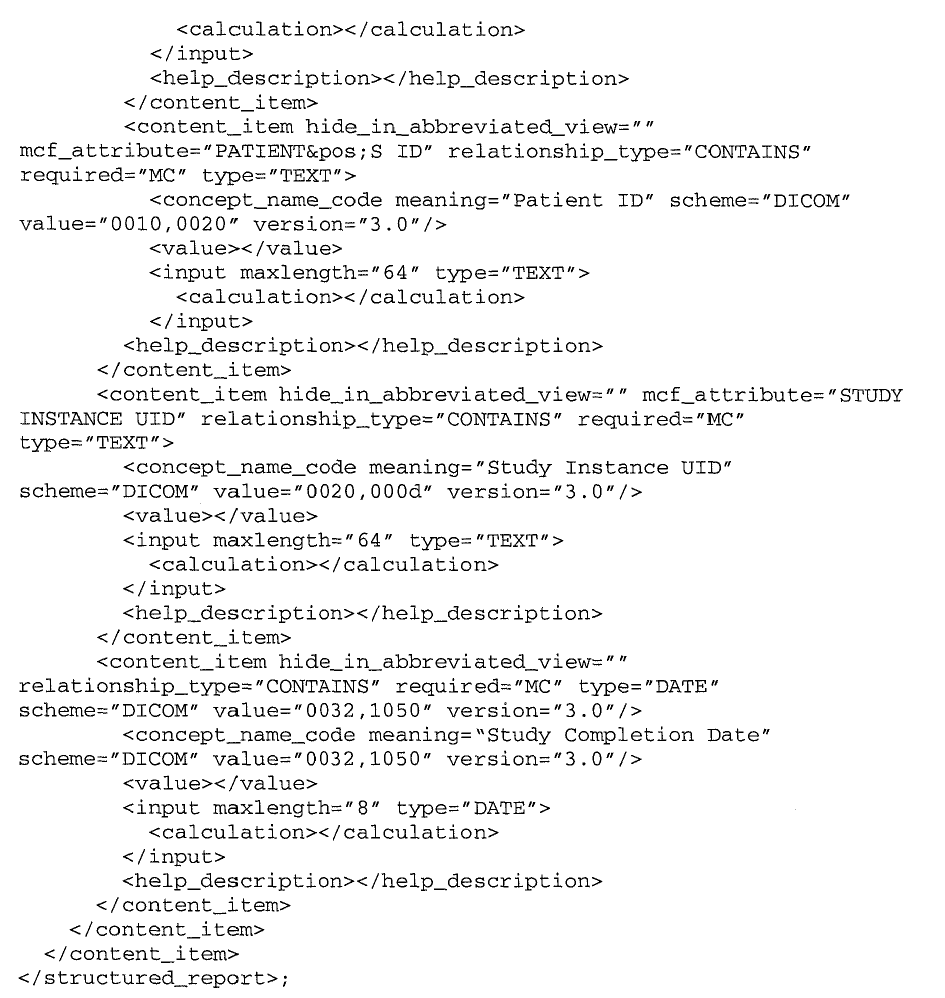 The root of the template is the "structured_report" element that contains all of the "content_item" elements for the report. As previously described, a "content_item" element can contain child "content_item" elements. In the above template example, the root "content_item" element for the report template is a "container" element named "Example." The "Example" element contains another "container" element named "Findings." The "Findings" element contains the remaining four "content_item" elements: "Patient Name," "Patient ID," "Study Instance UID," and "Study Completion Date."

When the template is saved to the SOR 42 or other storage location or device, the template may not contain all of the information concerning the concepts specified in the template. Information regarding the concepts may be separately stored in the SOR 42 or other storage location or device. The stored templates may reference the details or description for a concept contained in a template through a file or database stored in a separate location or device. The BLS 38 may be configured to reference specific information regarding the concepts listed in a template from a concept file or storage device and add the details to the report as required.

The template editor application or BLS 38 may be further configured to verify a new template or verify modifications to existing templates. Templates may require certain characteristics to ensure that the BLS 38 can utilize them to successfully generate reports. A template may need a unique name so that it can be properly identified. The templates may also require at least one "content_item." The logical statements included in the templates may also be verified.

Although a template has been generated, the BLS 38 may not be able to generate a report without being able to map the input data to "content_item" elements as described in the template. The data input by various input or procedure devices may arrive at the SR application 22 in a number of formats. Each received data set may be mapped into a format the BLS 38 can use. In some embodiments, mapping the input data into data understood by the BLS 38 is the responsibility of the inbound message devices 30 and 32.

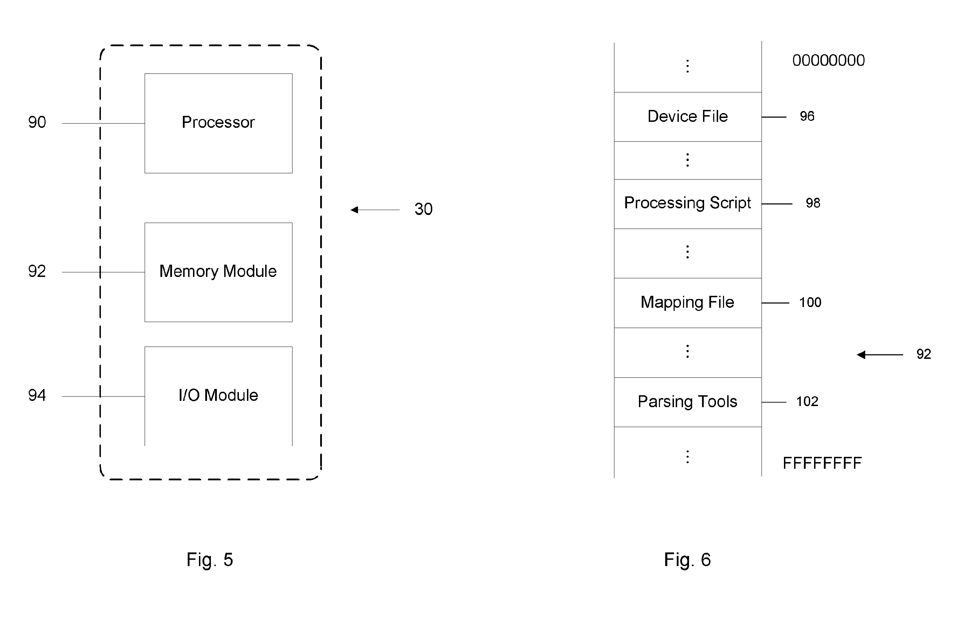

Fig. 5 illustrates the hardware components of an exemplary inbound message device 30. In the exemplary configuration shown, the inbound message device 30 includes a processor 90, a memory module 92, and an I/O module 94. The memory module 92 may contain nonvolatile memory, such as one or more forms of ROM; one or more disk drives; RAM; other memory; or combinations of the foregoing. The I/O module 94 may be configured to receive inbound messages from input devices such as the HIS 24 or hemodynamics server 26 and transmit messages to other components of the system 20.

Fig. 6 illustrates the possible contents of the memory module 92 or a portion thereof. As illustrated in Fig. 6, four components are stored in the memory module 92 including a device file 96, a message processing script or processing script 98, a mapping file 100, and parsing tools 102. In various implementations, the memory module 92 may be configured in such a way that it does not include four distinct portions. Functional features can be combined in a variety of ways. The file may also be located at other storage locations and transferred to the inbound message device 30 as requested or needed.

The inbound message device 30 may include multiple device files 96 and may include a device file 96 for each device that it receives messages from. Each device file 96 specifies data regarding an input device. An exemplary device file 96 is set out below. 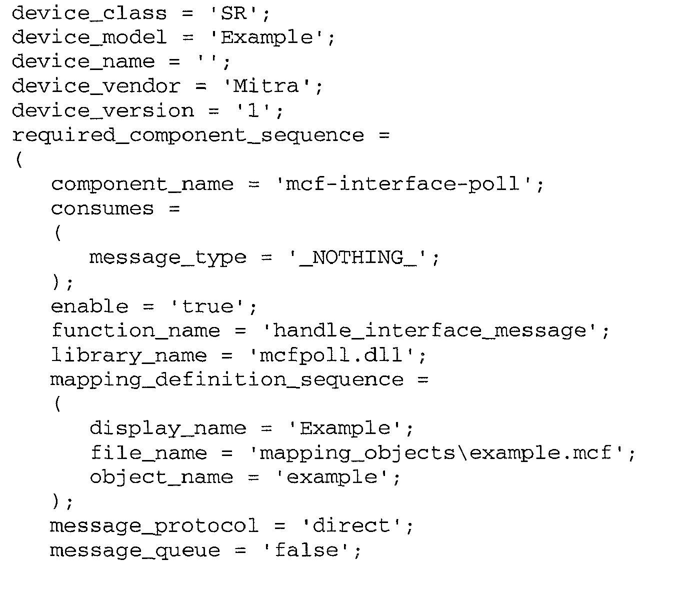 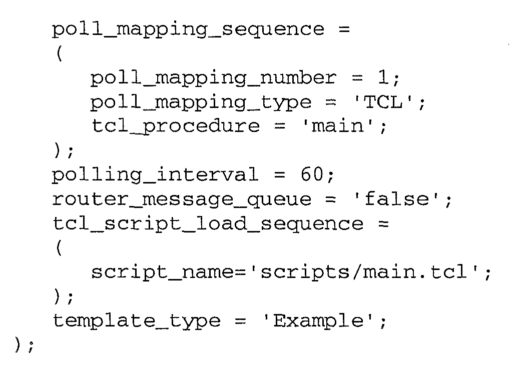 The device file 96 may include a poller component configured to determine whether the inbound message device 30 has received a message from the corresponding device. The device file 96 may also include a list of messages that are consumed or recognized by the device. The I/O module 94 may include an internal memory or buffer, or interact with a remote storage device, where received messages are stored until processed. The poller component of the device file 96 may query the I/O module 94 for received messages or may watch for messages to be stored in specific areas of a storage device. The poller component may be regulated by a polling interval specified in the device file 96. In the exemplary device file 96 above, the poller component is configured to check for messages every sixty seconds.

The messages received by the inbound message device 30 may include data to be placed in a report or may specify a location, such as a file name, where data can be found. The message may also provide notification or instruction. For example, the HIS 24 may generate a message indicating the scheduling of a procedure for a patient. The inbound device 30 receiving the message may pass the message on to the BLS 38 so that the BLS 38 can gather past reports or general data for the patient from internal or external storage devices. The BLS 38 gathers the data so that it can be used later to generate a report once the procedure is performed.

The device file 96 may also include a link or name of the template where data received from the device should be placed. The template link may be used by the inbound message device 30 to indicate which template to use in an instruction or message to the BLS 38 to generate a report. The template link may also be used by the template editor application. When a user wishes to update a template, the template editor application may present the user with a list of devices. The user may select a device from the list and the template editor may use the device file 96 to determine the template corresponding to the selected device.

Once the poller component has determined that a message or input file from the device corresponding to the device file 96 has been received, the poller component may be further configured to determine if the received message requires processing or if the message can be ignored. As previously described, the device file 96 may include a list of messages or files that are consumed or recognized by the poller component. The poller component may also specify actions to take when certain messages are received from the device. When the poller component determines that a message has been received that requires processing, the poller component may call a processing script 98. The poller component may pass the received message to the processing script 98 or may allow the processing script 98 to obtain the message from the I/O module 94 or specified memory location independently. The message or input data may include global objects that can be accessed by multiple components or applications.

The processing script 98, which knows the format of the input data from the device, generates a list or sequence of data items from the messages or input data sent by the device. An exemplary processing script 98 is set out below.

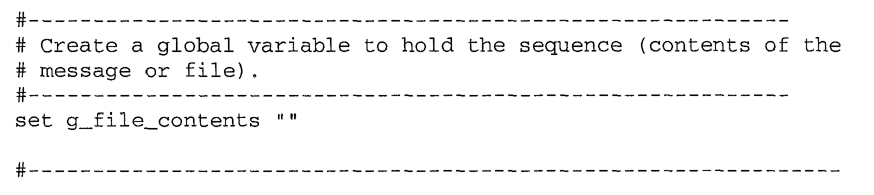

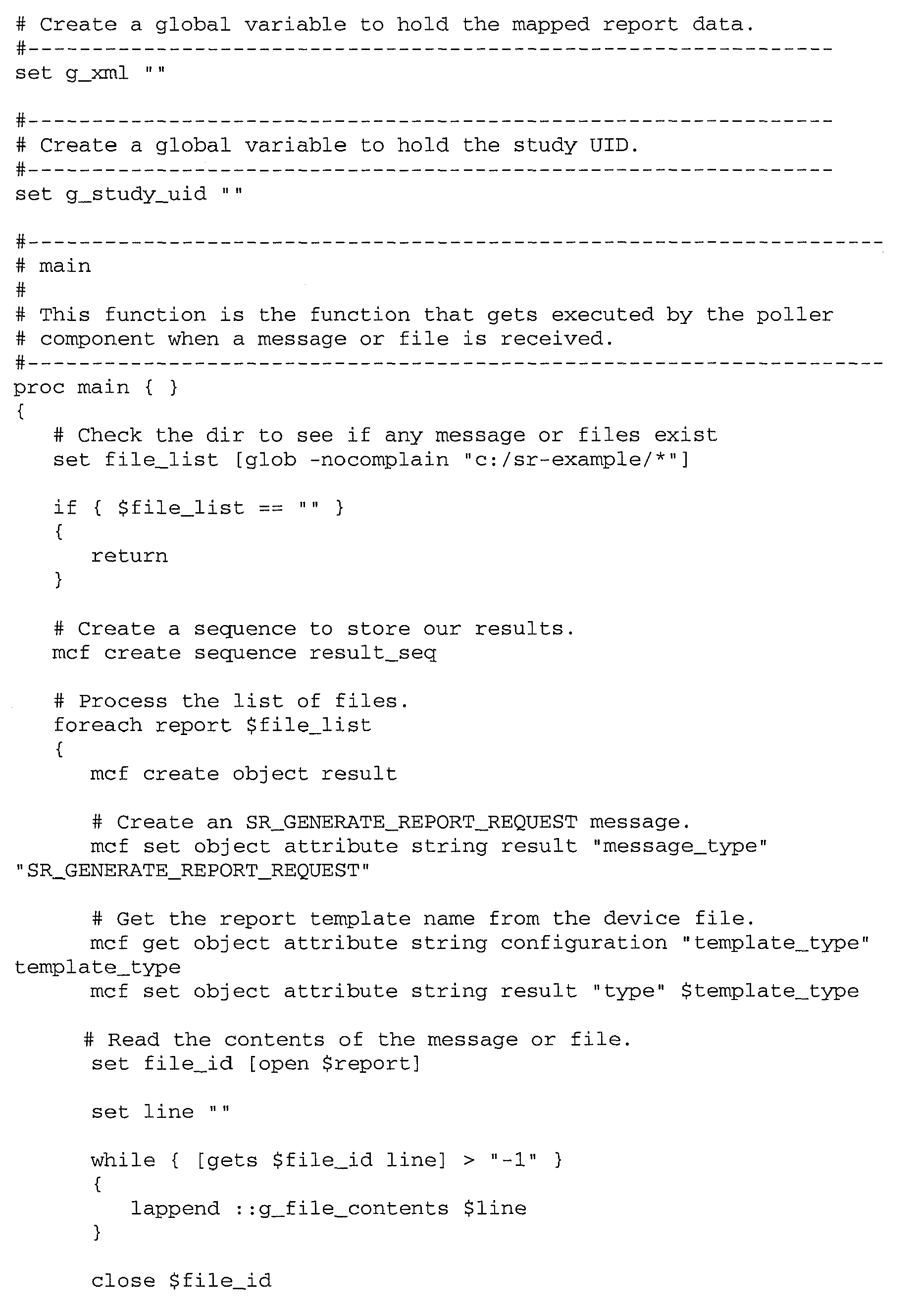

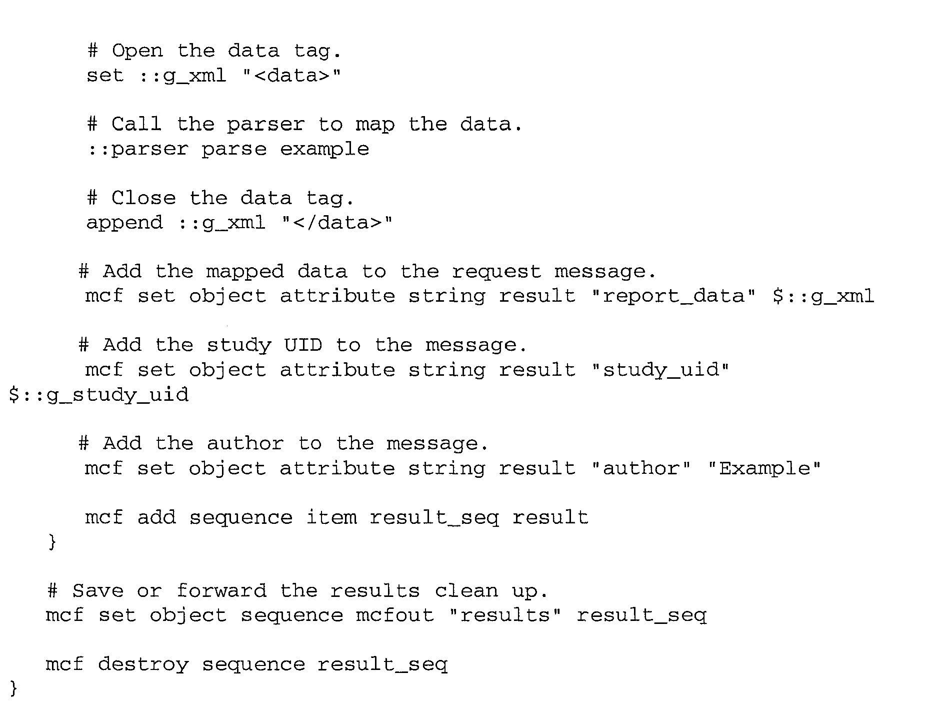

The processing script 98 may also supply other information in the sequence such as details of the message or file names. The sequence may take the form of a data stream with a known format that can be utilized later to parse the data. The sequence may also take the form of a file. For example, the processing script 98 may place each data item or concept value on a separate line in a file. When the data is later parsed, each line from the file can be read and mapped. If multiple messages or input files are received, the processing script 98 may include all the message data in a single sequence or may create multiple sequences, one for each message or input data set.

After the sequence of concept values has been created, the processing script 98 calls a parser. The parser may be part of the parsing tools 102 section of the memory module 92. The inbound message device 30 may include multiple parsers and may include a parser for each device that it receives messages from. In some embodiments, the parser may be configured to break the input data contained in the sequence into distinct data items. The parser may then map each data item or concept into a format understood by the BLS 38. The parser may access the mapping file 100 or data dictionary to perform the mapping. An exemplary mapping file 100 is set out below.

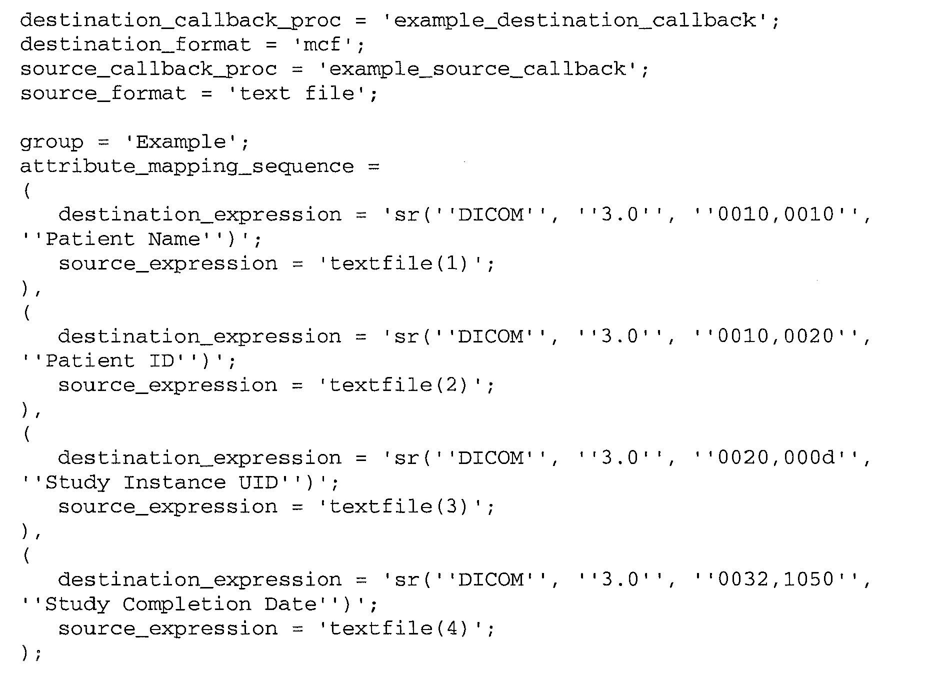

The mapping file 100 may specify the source expression or the location in the generated sequence, and the corresponding destination expression, code, or placeholder in the template. For example, the parser may read the first element in the sequence or the first line of the file and use the mapping file 100 to map the first element to a code. The source expression may be "first element in the sequence" or "first line of the file" and the destination expression may be a code type data structure. As previously described, data is mapped into templates using code type data structures. The code type data structure applies a common definition to the data item so that it can be placed in a template. For example, if the first data item of an input message or file is "Doe^John^^," the mapping file 100 corresponding to the device that generated the message or input file may describe "Doe^John^^" as a code type data structure with the attributes "DICOM," "0010,0010," "3.0," and "Patient Name." The mapping file 100 provides the common description or code type data structure for the input data. The parser may use the mapping file 100 to generate a data item that includes the code type data strucutre and the actual value. For example, the parser may add XML tags to the mapped data to generate an XML string as shown below for the exemplary input "Doe^John^^."

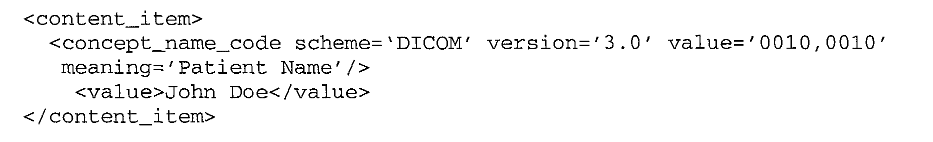

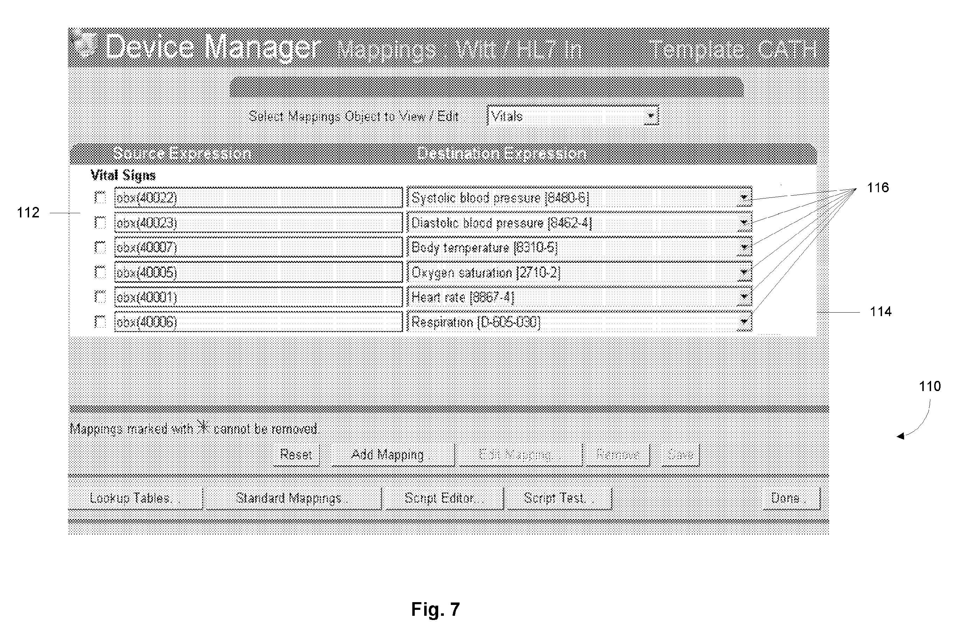

The mapping files 100 for input devices may be modified using a mapping editor application or device manager supplied by the service tools server 66. The mapping editor application displays the source expressions as listed in the mapping file 100 and allows each source expression to be mapped to a code type data structure. Fig. 7 illustrates an exemplary screen shot 110 from the mapping editor application. To edit a mapping file 100 a user may use the workstation 62 to interface with the service tools server 66 to execute the mapping editor application. To select a mapping file 100 to edit, the mapping editor application may list the devices that are known or registered with the SR application 22. In some embodiments, a device may be registered with the SR application 22 by having a device file 96. The user may select a device, and the mapping editor application may access the device file 96 for the selected device to determine the location of the mapping file 100. The mapping editor application may then present the user with editing screen 110 as shown in Fig. 7.

The screen 110 includes a source expression list 112 on the left and a destination expression list 114 on the right. The source expressions listed in the source expression list may be read from the mapping file 100 of the chosen device. The destination expressions may also be read from the mapping file 100 and listed in the destination expression list 114. Each destination expression may be listed as a descriptive string rather than a code type data structure. The descriptive strings help to reduce errors when selecting destination expressions because a user need not know or remember a code or type or otherwise input information to make a selection.

Each destination expression in the destination expression list 114 may include a drop-down list 116 or other selection mechanism that allows the user to select a new destination expression. The destination expressions listed in the drop-down list may include all possible destination expressions known by the mapping editor application. Alternatively, the drop-down list may only include destination expressions or codes listed in the template associated with the chosen device. Using the dropdown list may help avoid typographical and spelling errors. Such errors may lead to incomplete or erroneous reports, since such input data would be improperly placed in the template.

When used by the parser, the mapping file 100 aids the parser in generating XML strings that map the received data into data acknowledged by the BLS 38. The XML string generated by the parser may be returned to the processing script 98 where a "generate report request" is created to instruct the BLS 38 to generate a report from the mapped data. The message may include the mapped data, a template name to be used for the report, an author name, the study instance UID of the data, or the like. The data contained in the message may be used by the BLS 38 to name and store the completed structured report. For example, completed structured reports may be indexed in the SOR 42 or other storage device by study instance UID, date, patient name, or any combination thereof. The BLS 38 may be configured to name the completed structured report file with the study instance UID specified in the message or other unique identifier that will allow the completed report to be located when requested. The inbound message device 30 may communicate with the BLS 38 using a number of protocols. In some embodiments, the inbound message device 30 may use a proprietary message protocol such as the MCF protocol or another protocol designed to send sequenced attribute/value pairs to the BLS 38.

As previously described, upon receiving a "report generation request" and input data from the inbound message device, the BLS 38 retrieves the report template as specified in the report generation request from the SOR 42. In some embodiments, the BLS 38 may be configured to determine the report template to use without having to be explicitly instructed. The report templates define the contents of a structured report.

After the BLS 38 has acquired the template for the report it needs to generate, the BLS 38 is configured to place the received data into an instance of the template, which will become the report, by matching the code type data structure indicated in the template instance to the code type data structure matched with each data item. For example, the BLS 38 may receive the following string from one of the inbound message devices 30. 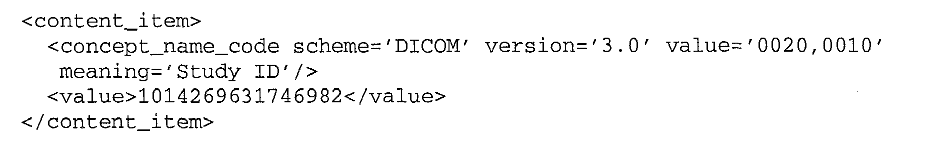 The BLS 38 may then attempt to find a matching element in the template instance. Matching elements have the same code type data structure. Taken from the exemplary template above, the following "content_item" element matches the received data string since they both include the same code type data structure. 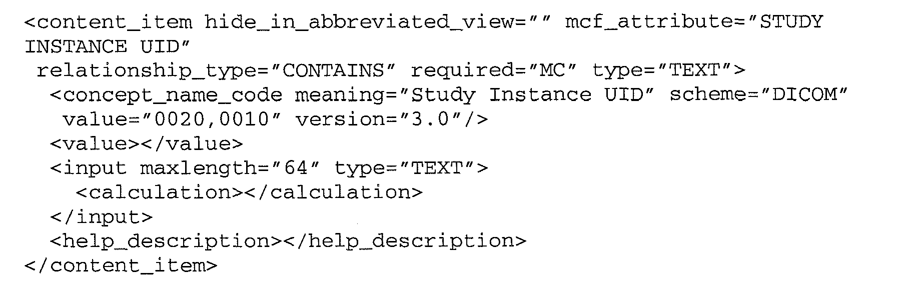 Upon matching the received string to an element in the template instance, the BLS 38 may be configured to take the value of the "value" component or element (1014269631746982) from the received data string and place the value into the empty "value" component as presented in the template instance to create an element for the final report.

Also, as previously explained, since report templates are based on a single XSD, the BLS 38 may obtain data from previous reports and place the obtained data into a new report. After the BLS 38 has placed the input data into the template instance, the generated report is saved to the SOR 42. The generated report is then viewable to a user through a report viewer application or an Internet browser. The stored generated report can also be exported and/or converted to other storage devices and/or systems as described above.

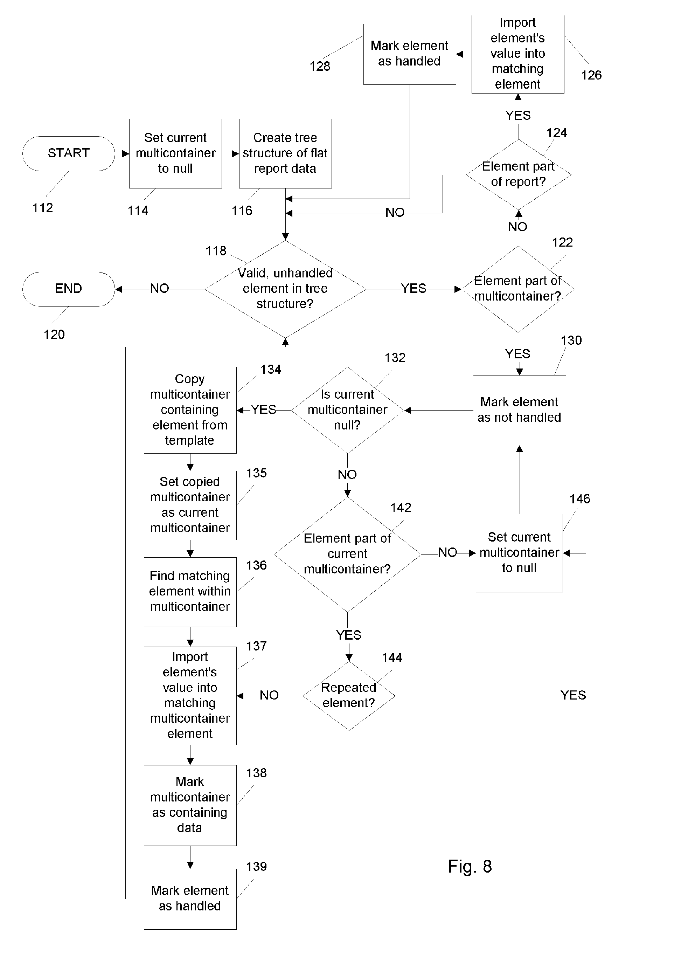

Fig. 8 illustrates an exemplary process for populating a report template with report data. The report template used to generate a report may represent a hierarchically structured report, in the sense that the report indicates relationships between data items. The report data supplied by external devices, such as an echocardiogram cart, may be flat data that does not specify relationships between data items. In some embodiments, the BLS 38 is responsible for converting the flat report data structure into a hierarchical structure.

As seen in Fig. 8, in some embodiments, upon receiving report data from an external device through the inbound message device 30 or directly, the BLS 38 starts the conversion process at block 112. Proceeding to block 114, the BLS 38 sets internal variables used during the conversion including a current multicontainer variable. Initially, this variable is set to null. As previously described, a multicontainer element may contain nested content_item elements 69. The multicontainer elements present in a report template specify relationships that provide a hierarchical structure to the report.

After setting the current multicontainer to null, the BLS 38 creates a document object model ("DOM") of the flat report data received from the external device (block 116). In some embodiments, the generated DOM is an XML tree structure DOM. Since the flat report data has little or no hierarchical structure, the resulting DOM may also have a flat structure such as a linear tree structure of data elements. After generating the DOM, the BLS 38 attempts to find a valid element in the DOM (block 118). In some embodiments, a valid element is indicated by an XML tag that is recognizable by the BLS 38. For example, an XML tag <content_item> may represent an opening tag of a valid element. Unrecognizable tags or unmarked text strings, numeric data, or white space may be ignored by the BLS 38. If the BLS 38 does not find a valid element in the DOM, or if the BLS 38 has already handled all the valid elements, the BLS 38 ends the conversion process (block 120).

If the BLS 38 finds a valid element, the BLS 38 determines if the element is part of a multicontainer element (block 122). In order to do so, the BLS 38 may generate a list of content_items elements 69 referenced in the report template that the BLS 38 is attempting to populate with the elements of the report data received from the external device. The BLS 38 may also create of list of multicontainers that the element is a part of. Initially, the list may be empty.

In some embodiments, the BLS 38 walks the list of content_item elements 69 of a report template and looks for a content_item element 69 that matches the element from the DOM. A content_item element 69 may have a matching coding scheme, coding scheme version, and coding value as the DOM element in order for the elements to be considered "matching" elements. If the BLS 38 finds a matching content_item element 69 in the list, the BLS 38 looks at an ancestor of the content_item element 69 to determine if the content_item element 69 is part of a multicontainer. If the matching content_item element 69 is part of a multicontainer, the BLS 38 adds the multicontainer to the list of found multicontainers. The BLS 38 may continue to look for other matching content_item elements 69 since a content_item element 69 may belong to a number of multicontainers. After the BLS 38 has exhausted the list of content_item elements 69, the BLS 38 examines the list of found multicontainers. If the list is empty, the BLS 38 has determined that the element is not part of a multicontainer. The BLS 38 then proceeds to determine if the element is a part of the report template (block 124). The BLS 38 may follow a similar method as performed when searching for multicontainers containing the element, but the BLS 38 may look for matching content_item elements 69 in the list without determining if they are part of a multicontainer or not. The BLS 38 imports the element's value into each matching content_item element 69 it finds (block 126). After importing the element's value into matching content_item elements 69, the BLS 38 marks the element as handled (block 128) and returns to look for another valid and unhandled element in the DOM (block 118). If a matching content_item element 69 was not found for the current element, the BLS 38 ignores the current element and proceeds to find another valid and unhandled element (block 118). In some embodiments, the BLS 38 may mark the unmatched element as handled or ignored so that the element is not processed again unnecessarily.

If, however, after walking the list of content_item elements 69 searching for multicontainers containing matching content_item elements, the list of found multicontainers is not empty, the BLS 38 concludes that the element is part of at least one multicontainer. The BLS 38 then marks the element as not handled (block 130) and determines whether the current multicontainer is null (block 132). In order to import a value for a content_item element 69 that is part of a multicontainer, the entire multicontainer must be imported. If the current multicontainer is null, each found multicontainer containing the matching content_item element 69 is copied from the template (block 134). As each multicontainer is copied into the template, it is set to the current multicontainer (block 135), and the matching content_item element 69 within the multicontainer is found (block 136). Once the matching content_item element 69 is found, the BLS 38 imports the element's value into the matching content_item element 69 (block 137). (Since a multicontainer may be repeatable and subsequent, similar element values from the DOM should be contained within a new multicontainer rather than overriding the previous values.) The BLS 38 marks the multicontainer as containing data at block 138. The BLS 38 then marks the element as handled (block 139) and returns to process another element at block 118.

If, however, the current multicontainer is not null, the BLS 38 determines if the element is part of the current multicontainer (block 142). The BLS 38 may compare the value of the current multicontainer variable to each multicontainer contained in the list of found multicontainers previously constructed. If the BLS 38 finds that one of the multicontainers listed in the found multicontainer list matches the current multicontainer, the BLS 38 determines if a value for the matching content_item element 69 within the multicontainer has already been imported from a previously processed element (block 144). If so, the BLS 38 concludes that the element is a repeated value and that another multicontainer must be added to hold the current element's value. The BLS 38 sets the current multicontainer to null (block 146) and marks the current element as unhandled (block 130). The BLS 38 then returns to block 132 where it determines if the current multicontainer is null, which it is, and proceeds to copy in another multicontainer to hold the current element's value.

If, however, the element is part of the current multicontainer and a value has not been previously imported for the matching content_item element 69 contained within the current multicontainer, the BLS 38 imports the element's value into the matching content_item element 69 (block 137). As previously described, the BLS 38 continues by marking the multicontainer as containing data (block 138) and marks the element as handled (block 139). Having handled the element from the DOM, the BLS 38 returns to block 118 to determine if there is another element in the DOM to process.

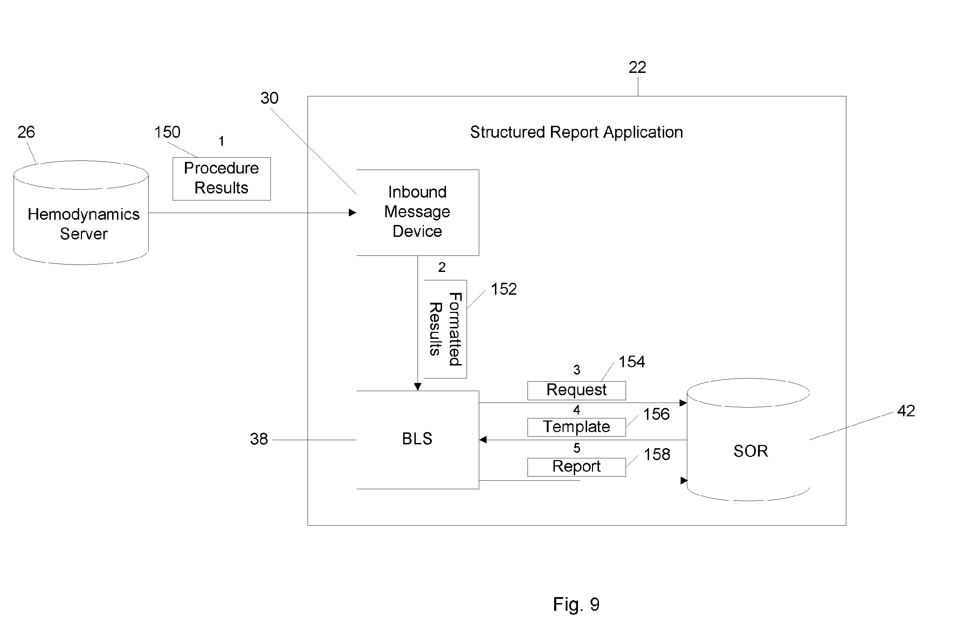

Figs. 9-13 illustrate exemplary interactions and data flow between components of the system 20. Fig. 9 illustrates the process of creating a structured report from results transmitted by an input or procedure device such as the hemodynamics server 26. In some embodiments, the first step of the process includes an input or procedure device, such as the hemodynamics server 26, generating a procedure results message 150 containing the results of a completed procedure. The results message 150 may be an HL7-formatted message, an HTTP-formatted message, or the like.

The results message 150 is received by an inbound message device 30, which determines the input or procedure device that generated the message and what the message includes. As previously described, the inbound message device 30 may be configured to map the data received in the results message 150 to data understood by the BLS 38. In a second step of the process, after mapping the data, the inbound message device 30 transmits the mapped results in a formatted results message 152 to the BLS 38. The formatted results message 152 may include a request to generate a structured report from the transmitted data. The formatted results message 152 may also include additional data that the BLS 38 may use to generate a structured report from the transmitted data. For example, the formatted results message 152 may include the name of the template the BLS 38 should use for the report. In a third step of the process, the BLS 38 may send a template request message 154 to the SOR 42 requesting the template specified in the formatted results message 152. Upon receiving the template request message 154, the SOR 42 may return an instance of the template to the BLS 38 in a template message 156 in a fourth step of the process.

The BLS 38 may then generate the structured report by placing the mapped or formatted data received in the formatted results message 152 from the inbound message device 30 into the instance of the template received from the SOR 42 in the template message 156. Finally, in a fifth step of the process, the BLS 38 sends the completed structured report to the SOR 42 in a report message 158 where it is stored by the SOR 42. As previously described, the inbound message device 30, BLS 38, and SOR 42 may utilize an attribute/value pairs messaging protocol such as the MCF protocol to communicate and send messages.

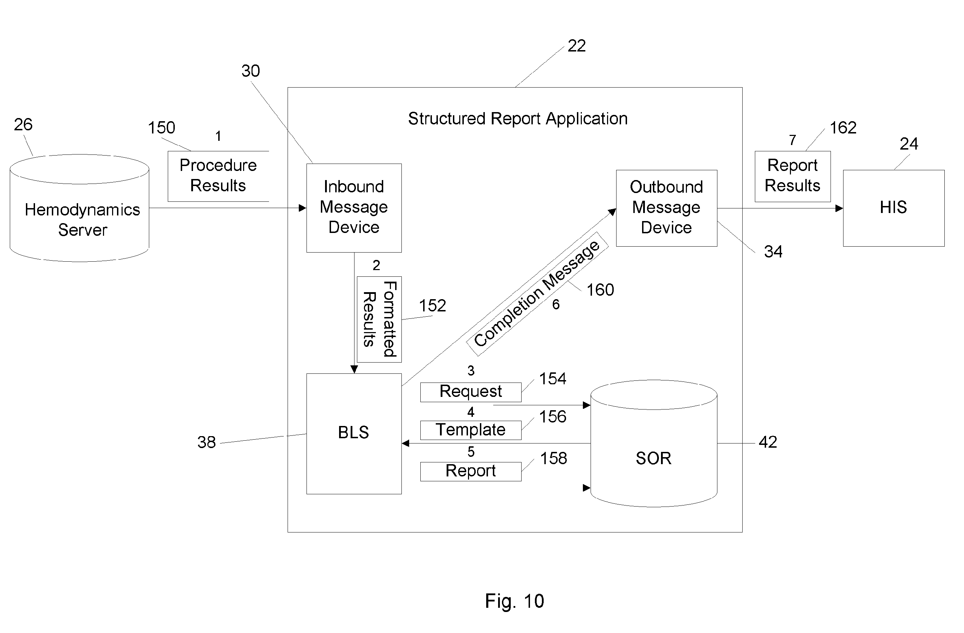

Fig. 10 illustrates the process of notifying an external device, such as the HIS 24, of the completed state and results of a structured report. The first five steps of the process are identical to those described for Fig. 9 and, therefore, will not be repeated here. In a sixth step, after the BLS 38 has stored the completed structured report to the SOR 42, the BLS 38 generates a completion message 160. The BLS 38 may transmit the completion message 160 to a number of devices or components including the outbound message device 34. The completion message 160 may state the fact that the report has been completed and may specify the file name or location of the completed structured report. The completion message 160 may also include the completed report or a portion thereof. The outbound message device 34 may use the data contained in the completion message 160 to generate a report results message 162. The outbound message device 34 may also use the data contained in the completion message 160 to gather data for the report results message 162 directly from the generated report. In a seventh step of the process, the outbound message device 34 may transmit the report results message 162 to an external device such as the HIS 24. The report results message 162 may be sent in an HL7 format or other format recognized by the external device receiving the message. The external device may use the report results message 162 to notify other components, execute other related applications such as a billing application, move or copy the stored structured report to another storage location, or may log the status of the structured report as complete.

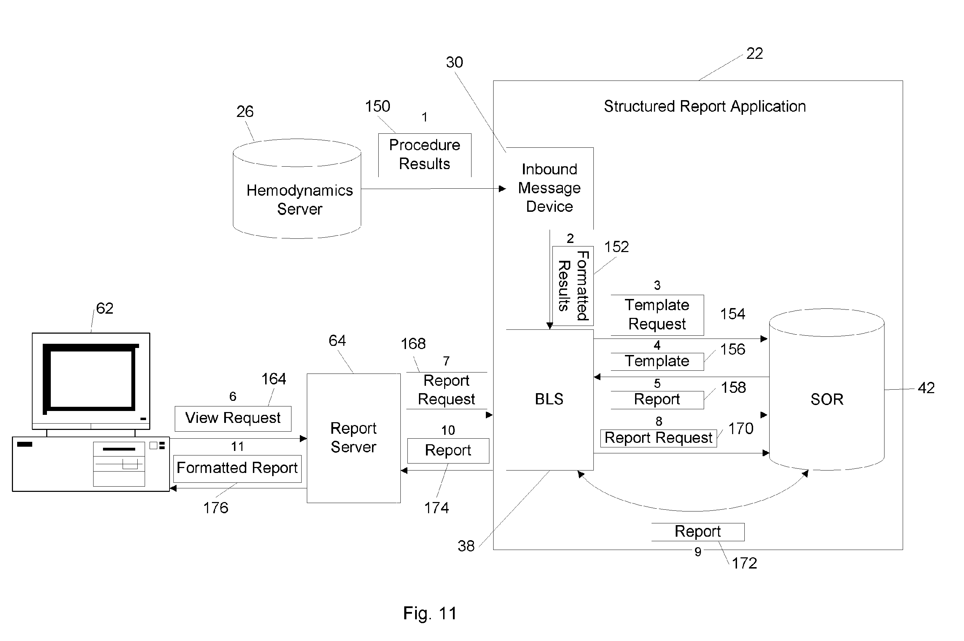

Fig. 11 illustrates the process of viewing a completed structured report. As in Fig. 10, the first five steps of the process are the same as in Fig. 9. In step six, after the BLS 38 has stored the completed structured report in the SOR 42, a user may request to view the completed structured report. The user may use a workstation 62 or the like to generate a report view request message 164. The user may generate the view request message 164 using a form displayed on the workstation 62 sent by the report server 64, or a report viewing application executing thereon. As previously described, the workstation 62 may communicate with the report server 64 using the HTTP protocol, although other protocols may also be used. The view request message 164 may include a unique identifier for the requested report or a set of identifiers including a study instance ID, a patient name, date, or any combination thereof. The user, or workstation 62, transmits the view request message 164 to the report server 64. In a seventh step, the report server 64 forwards a report request message 168 to the BLS 38. The BLS 38, in an eighth step, queries the SOR 42 by sending a second report request 170. The second report request 170 may be identical to the report request 168 sent by the report server 66. The BLS 38 may also create a second report request 170 specifically formatted for the SOR 42 from the data contained in the report request message 168.

Using the data specified in the second report request 170, the SOR 42 locates the requested report and returns the report in a report message 172 to the BLS 38 in a ninth step of the process. After receiving the report message 172, the BLS 38 transmits a second report message 174 to the report server 64 in step ten of the process. The second report message 174 may be identical to the report message 172 the BLS 38 received. The BLS 38 may also specifically format the second report message 174 for the report server 64. Finally, in an eleventh step of the process, the report server 64 transmits a formatted report message 176 to the workstation 62. The report server 64 may format the report by applying a stylesheet to the report or convert the report into a format recognized by the workstation 62 such as a PDF document. Upon receiving the formatted report message 176 from the report server 64, the user may view the completed structured report on a display of the workstation 62. Alternatively, the workstation 62 may be configured to format the report message 176 received from the report server 64 before displaying it. In some embodiments, the workstation 62 rather than the report server 64 may be configured to store, access, and apply a stylesheet to the formatted report message 176.

The user may also be able to use the workstation 62 to modify the displayed report. If the user is allowed to modify the report, the modified report may be sent back to the BLS 38 so that the modified report can be properly saved to the SOR 42. Modified reports may become versions of the original report or may override the original report.

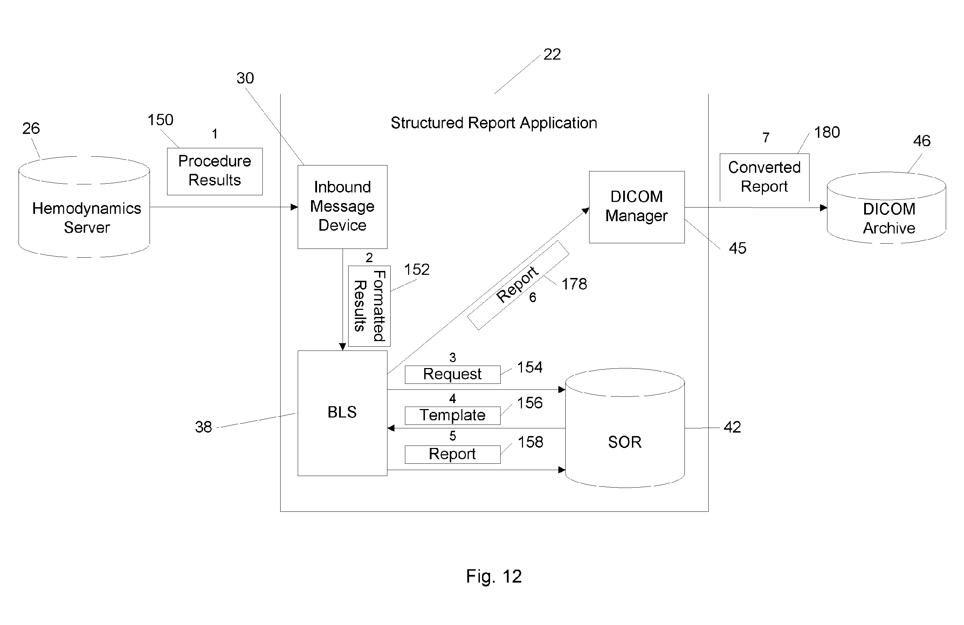

Fig. 12 illustrates the process of storing a completed structured report to an external storage location, such as the DICOM archive 46. As in Figs. 10 and 11, the first five steps of the process shown in Fig. 12 are the same as in Fig. 9. At a sixth step of the process, the BLS 38 sends a report message 178 to the DICOM manager 45. The report message 178 may be identical to the report sent to the SOR 42 for storage after generation. The report message 178 may also be formatted specifically for the DICOM manager 45. In a seventh step, upon receiving the report message 178, the DICOM manager 45 converts the report to a DICOM formatted report and sends a converted report message 180 to the DICOM archive 46. In some embodiments, the BLS 38 may be configured to perform the conversion to a DICOM structured report. The DICOM archive 46 stores the received DICOM report. The DICOM archive 46 may also perform additional formatting to the received converted report 180 before storing the report. As previously indicated, the DICOM archive 46 may be used for permanent data storage for completed reports. The completed structured report may be forwarded to other external systems following a similar process. In some embodiments, the BLS 38 may communicate with multiple managers, such as the DICOM manager 45, configured to receive BLS-generated structured reports and convert the received reports into vendor or system specific formats for storage, display, or editing purposes.

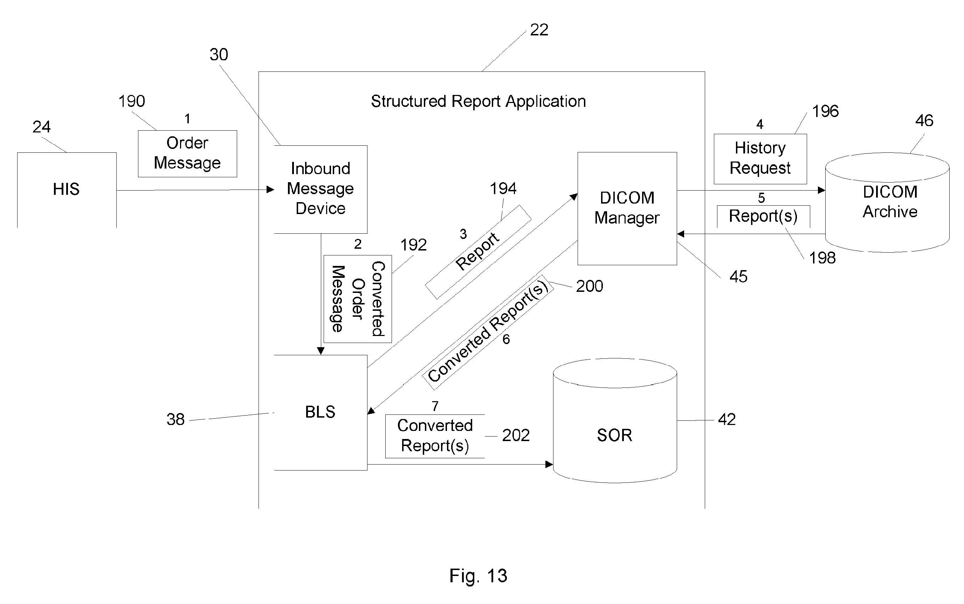

Fig. 13 illustrates a process that is opposite of the process depicted in Fig. 12. Fig. 13 illustrates the process of obtaining completed reports from external data storage devices such as the DICOM archive 46. The first step of this process includes the HIS 24 generating an order message 190. The order message 190 may indicate the scheduling of a procedure, the admittance of a patient, or the like. As previously indicated, the order message 190 may be transmitted as an HL7 message. The order message 190 may include data such as the patient name, patient ID, date of schedule procedure or admittance, or type of procedure scheduled. The order message 190 may also include historical data such as a list of past procedure results or admittance dates and durations.

At a second step of the process, the inbound message device 30 receives the order message 190 and converts the message 190 to a converted order message 192 recognized by the BLS 38. The inbound message device 30 may convert the order message 190 into an MCF formatted order message 192 or other message format understood by the BLS 38. The inbound message device 30 may also forward the order message 190 to the BLS 38 without formatting the message 190.

After converting or formatting the order message 190, the inbound message device 30 transmits the converted order message 192 to the BLS 38. Step three of the process includes the BLS 38 generating and transmitting a request 194 for the DICOM manager 45. The BLS 38 may communicate with the DICOM manager 45 using a DICOM messaging protocol, although other messaging protocols are possible. The request 194 may request historical information regarding the patient's past procedures and results.

Upon receiving the request message 194, the DICOM manager 45 may send a history request 196 to the DICOM archive 46 at a fourth step of the process. The history request 196 may request past reports related to the data indicated in the order message 190 sent by the HIS 24. For example, the DICOM archive 46 may return all past reports stored for the patient recently scheduled for a procedure by the HIS 24. The BLS 38 may incorporate the data from past report(s) into a new report that may be generated for the scheduled procedure.

At step five of the process, the DICOM archive 46 returns any related reports requested by the history request 196 to the DICOM manager 45 in a DICOM report message 198. Since the reports were stored in the DICOM archive 46, the returned reports may be DICOM-formatted reports. The DICOM manager 45 may convert the report message 198 containing the past report data into a format recognized by the BLS 38. Alternatively, the BLS 38 may be configured to convert the report received from the DICOM archive 46 rather than the DICOM manager 45.

The DICOM manager 45 sends the BLS 38 the converted report(s) in a converted report message 200 at step six of the process. The BLS 38 then forwards the converted report(s) to the SOR 42 in a second converted report message 202 in step seven of the process. Alternatively, the BLS 38 may forward the converted report message 160 to the SOR 42 without formatting the data of the converted report message 200. As previously stated, the BLS 38 may be configured to convert or format the reports received from external systems or storage devices. The BLS 38 may also be configured to provide additional formatting of the returned reports before sending the reports to the SOR 42 for storage. The reports and data received from external storage devices or locations may also be already in a format recognized by the BLS 38 and may not require formatting or conversions. Since the past report data has been moved to the SOR 42, the BLS 38 can access and use the data in a new report. A user may also view the transported report using a report viewing application as described in Fig. 11.

It should be understood that the components shown in Figs. 9-13 represent exemplary configurations. Additional components or multiple components of those shown may be added. Components may also be combined or broken down into separate components. For example, the functionality of the inbound message devices 30 and 32 may be included in the BLS 38 and implemented entirely in software and input or procedure devices may transmit messages to the BLS 38 directly rather than indirectly through the inbound message devices. The functionality of the BLS 38 and SOR 42 may also be combined.

The inbound message devices 30 and 32 may also be broken down into multiple components including a message buffer, a parser, a mapping device, or the like. Components such as the report server 64 may also be removed from the system 20. The workstation 62 may be configured to directly interface with the BLS 38 or other device of the SR application 22. The BLS 38 may also include the functionality of the DICOM manager 45, or other external system or device manager.

As previously described, departments, such as cardiology departments, can use many different devices in the treatment of patients. These devices export large amounts of data, such as clinical reports approved and/or transcribed by a physician and/or specialists of a medical system. Generally, there are few, if any standards governing the content or format of this data. This can make it difficult to process the data, such as by generating a structured report from the data, performing statistical analysis on the data, and/or mining the data.

As described above, the SR application 22, or, in particular, the inbound message devices 30 and 32, converts data from various formats into an internal format using a data dictionary, which can be represented in XML. The internal data dictionary can use a standard internal data format to represent various disparate data sets and can use a unique code to identify each data element. The unique code helps eliminate confusion about the contents of a data element. The internal data dictionary also helps medical systems to customize data in order to handle specific needs.

The BLS 38 generates a structured report from the converted data and stores the structured report to the SOR 42. A structured report can also be reformatted to an external format, such as a DICOM format and stored to an archive, such as the DICOM archive 46.

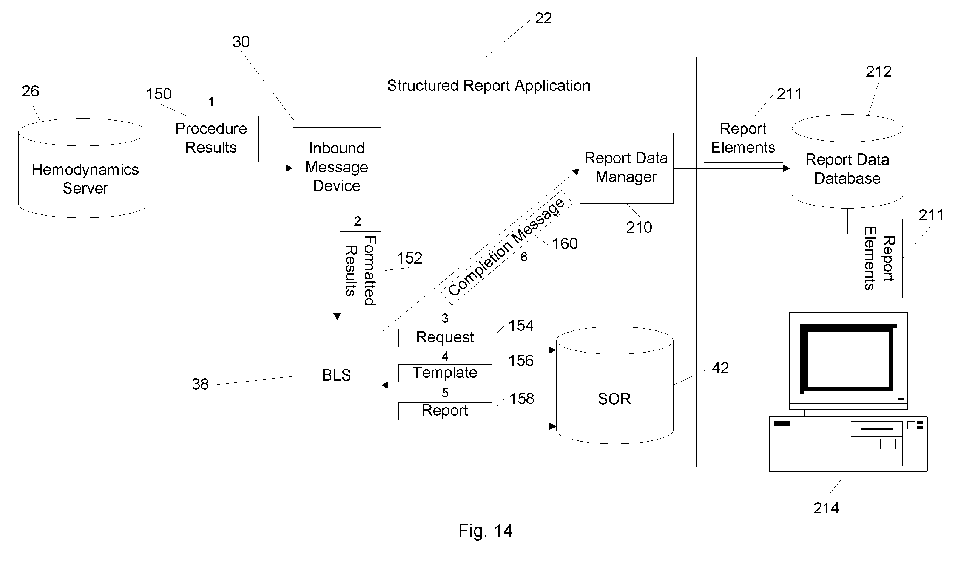

It may, however, be useful to generate various reports directly from clinical result data. In some embodiments, the SR application 22 includes a report data manager 210 that stores qualifying reports, such as structured report content, as report elements 211 to a report data database 212 (see Fig. 14). The report data manager 210 can consider any report that adheres to a particular format, such as a generalized structured report XML schema, as a qualifying report. Therefore, the report data manager 210 can obtain and process reports generated by systems and applications other that the SR application 22 as long as a report adheres to a format understandable to the report data manager 210. The report data manager 210 can be implemented as a system device in order to allow the enabling and disabling of the feature.

As described above with respect to Fig. 10, upon completing a structured report, the BLS 38 generates a "completion" message 160 indicating the creation of a new report. A "completion" message 160 can also indicate the approval and/or the transcription of clinical data and/or a corresponding structured report and may be generated by components other than the BLS 38. As also described, components and devices in the SR application 22 listen for "completion" messages and, upon receiving a "completion" message, export a structured report and/or related data to a secondary location.

As shown in Fig. 14, the report data manager 210 is configured to consume "completion" messages 160 sent from the BLS 38 and/or another component at the sixth step of the process of creating a structured report. In some embodiments, the report data manager 210 stores a device file, as described above with respect to Fig. 6, that specifies settings for the type of message that the report data manager 210 consumes. For example, the device file for the report data manager 210 can specify a system name and/or identifier of the report data manager 210, the type of messages the report data manager 210 consumes (e.g., "completion" messages, "SR_TRANSCRIBED" messages, and/or "SR_APPROVED" messages), a system name and/or identifier of the report data database 212, a login and/or password for the report data database 212, one or more libraries that the report data manager 210 accesses, and an enable/disable setting for the report data manager 210. The BLS 38 or another component, such as a message router, uses the device file to determine if messages generated within the SR application 22 (e.g., with the BLS 38) and/or with components external to the SR application 22 should be routed to the report data manager 210.

In some embodiments, a "completion" message 160 includes a structured report generated with the BLS 38. A "completion" message 160 can also provide data for retrieving a structured report from a separate location, such as the SOR 42. In some embodiments, the structured report associated with a "completion" message 160 includes XML data, and the report data manager 210 takes the XML data and decomposes the XML data into individual data or report elements 211. The report data manager 210 then stores the report elements 211 to the report data database 212 in database tables. In some embodiments, each unique report element 211 receives its own column in a database table of the report data database 212. The report data manager 210 can be configured to store report elements 211 to columns and tables in the report data database 212 with predictable names or identifiers. For example, the report data manager 210 can create column and table names based on identifiers or other information included in or associated with a report element. A user can then use a query engine 214 to query the report data database 212 in order to process report elements 211 (e.g., statistically analyze the data, mine the data, etc.). In some embodiments, the query engine 214 includes a reporting engine, such as a Crystal Reports® engine manufactured by Business Objects®, a Congos ReportNet® engine manufactured by Congos Incorporated®, and/or a Microsoft Standard Query Language ("SQL") Server Reporting Services® engine manufactured by Microsoft Corporation®.

In some embodiments, the data received by the report data manager 210 changes based on changes made to the structured reports templates from which the structured reports are generated. For example, the number, format, and/or types of report concepts contained in a structured report are configurable by modifying a structured report template. To account for changes to the number, format, and/or types of report concepts contained in a structured report, the report data manager 210 can automatically create and update database tables of the report data database 212 at run-time in order to hold report elements 211 of a structured report, which may change as report concepts contained in a structured report are modified. For example, as new report templates are created, the report data manager 210 creates new tables containing a complete set of columns where each column is associated with a report element 211 generated from a structured report. As additional report concepts are added to and/or modified in the same template, the report data manager 210 either creates new child tables or adds new columns to existing tables of the report data database 212. In addition, if the report data manager 210 receives a structured report that it has already decomposed into report elements 211 and the report elements 211 have been stored in the report data database 212 (i.e., the report data database 212 already includes an identifier associated with a received report), the report data manager 210 updates the tables of the report data database 212 based on re-received structured report in order to store only the latest version of a structured report. The report data manager 210 can also re-decompose a structured report into report elements 211 and re-store the report elements 211 in the report data database 212 as a second version of the structured report.

The report data database 212 includes database report tables generated to hold report elements 211 obtained from structured reports generated with the BLS 38. In some embodiments, the report data database 212 includes one or more SQL servers, such as those manufactured by Microsoft®. An external application (e.g., the query engine 214) is granted select permission on generated report tables and uses a link, such as an open database connectivity ("ODBC") link to access data stored in the report data database 212. A login and/or password can also be used in order to provide and regulate access to (e.g., select-only) the tables of the report data database 212. In some embodiments, data manipulation permissions for the report data database 212 remain limited to a login used exclusively by the report data manager 210.

In some embodiments, backup of the report data database 212 is available through a database manager, for example an Enterprise Manager of Microsoft® SQL Server software. The database manager can automatically perform data backup functions or provide a dialog that prompts a user for a data backup description and a destination (e.g., a disk or a tape). The dialog can also allow a user to set up an automated data backup schedule.

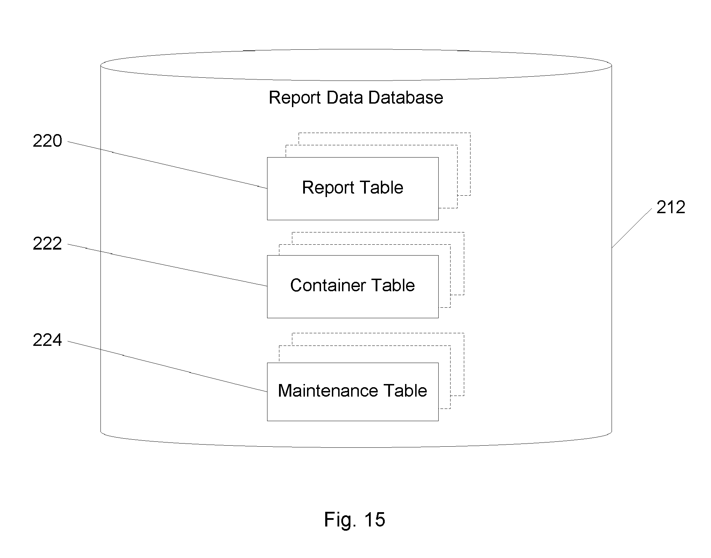

As shown in FIG. 15, the report data database 212 includes three categories of tables: report tables 220, container tables 222, and maintenance tables 224. In some embodiments, maintenance tables 224 do not contain specific report or patient information, but hold data for table maintenance and report generation.

Report tables 220 hold data regarding a particular structured report. In some embodiments, report tables 220 include two pieces of data which form a corresponding database object unique identifier. The identifier can take the form:
SRDD_<PARENT CONTAINER IDENTIFIER (if any)>_<CONCEPT MEANING IDENTIFIER>
where "CONCEPT MEANING IDENTIFIER" includes the code meaning of a top-level report concept or a version thereof (e.g., the first letter of each word found in the code meaning for a content_item element 69) and/or the code meaning of children report concepts or a version thereof. The placeholder "PARENT CONTAINER IDENTIFIER" includes a unique identifier of a current container where a report concept resides. In some embodiments, report concepts within the top-level of a container of a structured report do not have a "PARENT CONTAINER IDENTIFIER" component in a database object unique identifier. For example, if a report concept is a container that has no parent container and has a code meaning of "ECHOCARDIOGRAM MEASUREMENTS," the report table associated with the report container has a database object unique identifier set to "SRDD_EM," where the prefix "SRDD_" stands for structured report discrete data. As described above, the report data manager 210 can use the above naming convention to establish a set of predictable names or identifiers for columns and tables included the report data database 212 to store report elements. Using the predictable columns and tables identifiers, a user can query the report data database 212 for report elements without having to be explicitly informed of the structure of the report data database 212. The user can predict or determine the identifiers of the columns and tables included in the report database 212 based on known report concepts included in a structured report and a known naming convention used with the report data manager 210.

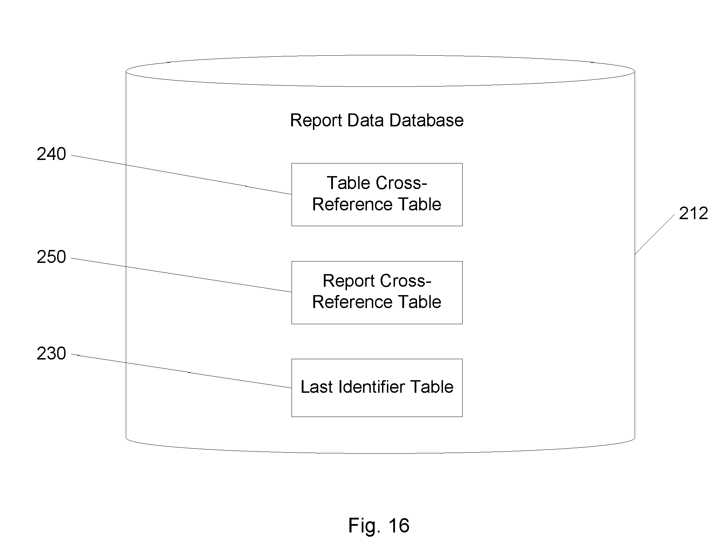

Each report table 220 and/or container table 222 can contain one primary key column whose name has the form:
i<ID_NAME>_ID
where "ID_NAME" is the name of the table without the "SRDD_" prefix. In some embodiments, the report data database 212 includes a last identifier maintenance table 230, as shown in Fig. 16, that maintains a running minimum value of available primary keys for a given table. Table 1 provides an exemplary structure for the last identifier maintenance table 230 (i.e., "SRRD_LAST_ID"). An additional column in the last identifier maintenance table 230 holds the latest code meaning of a report container for tracking purposes. Each container and/or report table can have exactly one row in the last identifier maintenance table 230.

**Table 1**

| SRDD_LAST_ID | | |
|---|---|---|
| column_name | Datatype | Comments |
| Id_name | varchar(128) | identifier name - table name, maint index |
| Id_value | Int | last used id value |
| Id_meaning | varchar(64) | meaning or description of id_name |

The report data database 212 can also include a container or table cross-reference maintenance table 240, as also shown in Fig. 16. Table 2 below illustrates an exemplary structure of a table cross-reference maintenance table 240 (i.e., "SRDD_TABLE_XREF"). The table cross-reference maintenance table 240 describes parent-child relationships within all container tables 222 for a report template. Each container table 222 in the report data database 212 is assigned a numeric table identifier (i.e., "table_id"), which is stored in a column of the table cross-reference table 240. The table cross-reference table 240 also stores a corresponding parent table identifier (i.e., "parent_table_id") that provides the numeric table identifier of a parent table of a container table 222. If a container table 222 has no parent table, the parent table identifier can have a value of zero or null. For example, top-level container tables 222 of a structured report do not have a parent container and, therefore, have a parent table identifier set to zero or null. The table cross-reference table 240 can also provide an exact primary key identifier of a container table 222 in order to aid the reporting and querying of report elements 211 stored in the report data database. The table cross-reference table 240 can also be used in the construction of comprehensive queries via stored procedures where related tables may need to be determined and accessed.

**Table 2**

| SRDD_TABLE_XREF | | |
|---|---|---|
| column_name | datatype | Comments |
| table_id | int | unique numeric identifier of table |
| table_name | varchar(128) | physical name of the table |
| table_meaning | varchar(128) | concept meaning of the top-level container |
| primary_key | varchar(128) | physical name of the primary key column |
| parent_table_id | int | numeric identifier of parent table |

The container cross-reference maintenance table 240 can be introduced in order to help eliminate the possibility of similar meanings of distinct structured report templates mapping to table name or identifier. For example, an "ECHOCARDIOGRAM MEASUREMENTS" template produces the same top-level table name (i.e., "SRDD_EM") as an "ELECTROENCEPHALOGRAM MEASUREMENTS" report. As shown in Fig. 16, the report data database 212 also includes a report cross-reference maintenance table 250 that associates a top-level table name with a specific report container scheme, version, and code value ("SVV") combination in order to further ensure that two structured report templates map to distinct table names. An exemplary report cross-reference maintenance table 250 (e.g., "SRDD_REPORT_XREF") is shown below in Table 3. Assuming the "EEG MEASUREMENTS" template has a unique SSV combination, the report data manager 210 recognizes that a table identifier "SRDD_EM" is already in use by a different report using the cross-reference maintenance table and/or the report cross-reference maintenance table 250. As shown in Table 3, the report cross-reference maintenance table 250 includes a report identifier (i.e., "report_id") that indicates a unique identifier for a report template that already uses the table name in question. When multiple report templates map to the same table name, the report data manager 210 uses the report identifier associated with the repeated name to construct a unique name. For example, the report identifier can be modified (e.g., incremented, decremented, doubled, etc.) and appended to the repeated table name to produce a unique name, such as "SRDD_EM_2." The new table name can then be added to the cross-reference maintenance table 240 and the report cross-reference maintenance table 250.

**Table 3**

| SRDD_REPORT_XREF | | |
|---|---|---|
| column_name | Datatype | Comments |
| report_id | Int | unique numeric identifier of report template |
| table_name | Varchar(128) | physical name of the top-level table |
| Scheme | Varchar(16) | top-level container concept scheme |
| Version | Varchar(32) | top-level container concept version |
| Code | Varchar(64) | top-level container concept code value |
| report_meaning | Varchar(128) | top-level container concept meaning |

In some embodiments, upon decomposing a structured report into one or more report elements 211, the discrete data manger 210 translates data types of report concepts contained within a structured report into column name prefixes prior to constructing a column identifier for a particular report concept. Column prefixes can include standard prefix notation, such as Microsoft® SQL prefix notation, which corresponds to a column's datatype. A column prefix based on a column's datatype assists third parties (e.g., the query engine 214) when creating database queries. A column prefix notation based on a column's datatype also forces the report data manager 210 to create a new column if a report concept included in a structured report changes its type. Table 4 provides datatypes and corresponding column prefixes using SQL prefix notation.

**Table 4**

| Concept Type | SQL datatype | SQL prefix |
|---|---|---|
| Code | varchar(128) | vch |
| Date | datetime (@12:00am) | dt |
| Datetime | Datetime | dt |
| Num | Float | f |
| Pname | varchar(64) | vch |
| Text | varchar(128) | vch |
| Time | varchar(16) (HH:MM:SS.SSSS) | vch |
| Image | varchar(128) | vch |
| (primary and foreign keys) | Int | i |

Unique column names of a table can be constructed with a container identifier, a concept code meaning, and a concept type/prefix translation using the following exemplary form:
<prefix ><CONTAINER ID (if any)>_<CONCEPT_MEANING>.
For example, a report concept representing a patient's age (e.g., meaning = "Patient Age") that has a numeric (i.e., "NUM") type and is included in a container identified by the name "DEMOGRAPHICS" is assigned a column name "fDEMOGRAPHICS_PATENT_AGE." Using Table 4, the "f" prefix represents a float datatype that corresponds with a report concept having a numeric type. Similarly, a report concept representing diastolic blood pressure (e.g., meaning = "Diastolic Pressure") that has a numeric type and is included in a container identified by the name "PATIENT_DATA," is assigned a column name "fPATIENT_DATA_DIASTOLIC_PRESSURE."

In some embodiments, a top-level report table contains three columns that uniquely identify a patient examination and its results ("vchREPORT_UID"), a study ("vchSTUDY_UID"), and a patient ("vchPATIENT_UID") instance. Values for these columns can be determined from a "report_uid" value, a "study_uid" value, and a "patient_uid" value that arrive as message attributes external to a structured report. A top-level table can also contain one or more "datetime" columns that store a last insert timestamp and/or a last update timestamp. In some embodiments, all columns other than the columns including the primary key and foreign keys allow NULL values. An exemplary top-level report table for an "ECHOCARDIOGRAM MEASUREMENTS" structured report is shown below in Table 5.

**Table 5**

| SRDD_EM | | |
|---|---|---|
| column_name | Datatype | comments |
| iEM_ID | Int | primary key id |
| vchREPORT_UID | varchar(128) | report sop instance uid |
| vchSTUDY_UID | varchar(128) | study sop instance uid |
| vchPATIENT_UID | varchar(128) | patient sop instance uid |
| dtINSERTED_DT | Datetime | record insert timestamp |
| dtUPDATED_DT | Datetime | record update timestamp |
| vchDEMOGRAPHICS_PATIENT_NAME | varchar(64) | patient name in demographics |
| fDEMOGRAPHICS_PATIENT_AGE | Float | patient age in demographics |
| fPATIENT_DATA_DIASTOLIC_PRESSURE | Float | diastolic blood pressure in patient data |
| fPATIENT_DATA_SYSTOLIC_PRESSURE | Float | systolic blood pressure in patient data |

In an attempt to simplify storing and querying for reporting purposes, a report concept that includes a units code (i.e., a content_item element 69 that includes an input_codes element 69d) also produces a units column in a report table that includes the column for the value of the report concept (i.e., the value element 69b of the content_item element 69 containing the report concept). In some embodiments, a units column can have a variable character string (varchar) datatype with a predetermined maximum string length (e.g., 24) that indicates a units label for the value of a report concept (e.g., inches, millimeters, beats per minute, etc.). In this way, as units codes change over time, a current unit code is stored along with a current value of the report concept (i.e., the value element 69b). In some embodiments, if a report concept does not include a valid units code, a corresponding units column is not produced. For example, a units column can be generated for each report concept that has a numeric type. New units columns can also be created as new report concepts are added and/or modified to a new and/or an existing report template. The units column naming convention can have the following form:
unit<CONTAINER ID (if any)>_<CONCEPT MEANING>.

An exemplary top-level report table for an "ECHOCARDIOGRAM MEASUREMENTS" structured report including units columns is shown below in Table 6.

**Table 6**

| SRDD_EM | | |
|---|---|---|
| column_name | Datatype | comments |
| iEM_ID | Int | primary key id |
| vchREPORT_UID | varchar(128) | report sop instance uid |
| vchSTUDY_UID | varchar(128) | study sop instance uid |
| vchPATIENT_UID | varchar(128) | patient sop instance uid |
| dtINSERTED_DT | Datetime | record insert timestamp |
| dtUPDATED_DT | Datetime | record update timestamp |
| vchDEMOGRAPHICS_PATIENT_NAME | varchar(64) | patient name in demographics |
| fDEMOGRAPHICS_PATIENT_AGE | Float | patient age in demographics |
| unitDEMOGRAPHICS_PATIENT_AGE | varchar(24) | units of patient age |
| fPATIENT_DATA_DIASTOLIC_PRESSURE | Float | diastolic blood pressure in patient data |
| unitPATIENT_DATA_DIASTOLIC_PRESSURE | varchar(24) | units of diastolic blood pressure |
| fPATIENT_DATA_SYSTOLIC_PRESSURE | Float | systolic blood pressure in patient data |
| unitPATIENT_DATA_SYSTOLIC_PRESSURE | varchar(24) | units of systolic blood pressure |

It should be understood when establishing table and/or column names or identifiers, some report concepts can include elements that include characters not included in a character set compatible (e.g., a SQL compatible character set) with the report data database 212 and, in some embodiments, incompatible characters are replaced with an underscore. For example, the report data database 212 may only allow table and/or column names to include characters within the ranges {A-Z, a-z, 0-9, _, space}. Characters outside the preferred range can be ignored or replaced when creating table and/or column names and not placed in a name.

In some embodiments, additional requirements are set on the characteristics used in table and/or column names in order to match a naming convention, such as the Microsoft® SQL naming convention. For example, a naming convention may require spaces to be converted to underscores and/or that names do not begin with a non-alphabetic character. Name length requirements may also be enforced and column and/or table names longer than a predetermined number of characters (e.g., 128 characters) can be automatically truncated to include only the predetermined number of characters (e.g., the right-most 128 characters).

A character encoding scheme of a structured report can also impose compatible and/or recommended characters. For example, if a structured report's character encoding scheme is ISO-8859-1 (Latin 1 encoding), additional character ranges are allowed in table and/or column names. In particular, the following additional characters are allowed: 0xC0 - 0xFF, except D7 (extended multiplication sign) and F7 (extended divide sign), 0xB5 (lower Greek mu), and 0x9F (dot-dot over Y).

As previously described, a structured report can have one or more containers and/or multicontainers that contain nested report concepts. A multicontainer element represents a tree of report concepts that is, in some embodiments, repeatable. Nested report concepts contained in the mulitcontainer tree indicate relationships between report concepts. Each multicontainer element can contain multiple child report concepts and can be repeated multiple times.

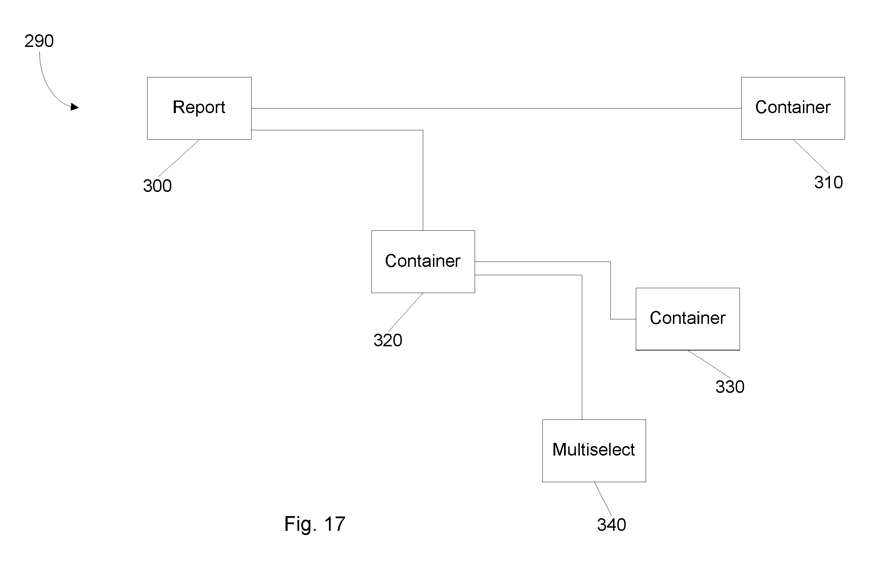

Since a multicontainer can have a one-to-many relationship with a structured report, an auxiliary table with proper container elements is created to hold report concepts included in multicontainers. A container table for a multicontainer includes a primary key identifier and also includes the primary keys of a parent record and one or more grandparent records. The container table can also store the meaning (i.e., identifier) of a possible parent single container element (non-table) in which a multicontainer resides. In the exemplary report structure 290 shown in Fig. 17, a report 300 includes two containers, a first container 310 and a second container 320. The second container 320 also contains its own child container 330 as well as a multi-select concept 340.

A multi-select concept is a special version of a container that holds data for multi-select code concepts. These concepts allow multiple code selections for a single concept. This one-to-many relationship can be stored as a standalone table with references to the parent tables.

The report structure 290 described and illustrated with respect to Fig. 17 can produce the following exemplary tables in the report data database 212 as shown in Tables 7 - 11.

**Table 7**

| SRDD_REPORT300 | | |
|---|---|---|
| column_name | datatype | Comments |
| iGR1_ID | Int | identifier for report record |
| vchREPORT_UID | varchar(128) | report identifier |
| vchSTUDY_UID | varchar(128) | study identifier |
| vchPATIENT_UID | varchar(128) | patient identifier |
| dtINSERTED_DT | datetime | record insert timestamp |
| dtUPDATED_DT | datetime | record update timestamp |
| <report specific columns> | | |

**Table 8**

| SRDD_REPORT300_CONTAINER310 | | |
|---|---|---|
| column_name | datatype | Comments |
| iGR1_CONTAINER_1_ID | Int | identifier for aux container 1 record |
| iGR1_ID | Int | identifier for report record |
| vchCONTAINER | varchar(128) | identifier of possible parent single container |
| <container 1 specific columns> | | |

**Table 9**

| SRDD_REPORT300_CONTAINER320 | | |
|---|---|---|
| column_name | datatype | comments |
| iGR1_CONTAINER_2_ID | int | identifier for aux container 2 record |
| iGR1_ID | int | identifier for report record |
| vchCONTAINER | varchar(128) | identifier of possible parent single container |
| <container 2 specific columns> | | |

**Table 10**

| SRDD_REPORT300_CONTAINER320_CONTAINER330 | | |
|---|---|---|
| column_name | datatype | comments |
| iGR1_CONTAINER_2_CONTAINER_3_ID | int | identifier for aux container 3 record |
| iGR1_CONTAINER_2_ID | int | identifier for parent aux container 2 record |
| iGR1_ID | int | identifier for report record |
| vchCONTAINER | varchar(128) | identifier of possible parent single container |
| <container 3 specific columns> | | |

**Table 11**

| SRDD_REPORT300_CONTAINER320_MULTISELECT340 | | |
|---|---|---|
| column_name | datatype | comments |
| iGR1_CONTAINER_2_MULTISELECT_1_ID | int | identifier for aux multi select record |
| iGR1_CONTAINER_2_ID | int | identifier for parent aux container 2 record |
| iGR1_ID | int | identifier for report record |
| vchCONTAINER | varchar(128) | identifier of possible parent single container |
| <multiselect 1 coded value column> | varchar(32) | code selection |

Using the above exemplary tables generated for the report 300, associated rows added to the last identifier maintenance table 230 are shown in Table 12.

**Table 12**

| SRDD_LAST_ID | | |
|---|---|---|
| ID_NAME | ID_VALUE | ID_MEANING |
| SRDD_REPORT300 | 2 | "REPORT 300" |
| SRDD_REPORT300_CONTAINTER310 | 4 | "CONTAINER 310" |
| SRDD_REPORT300_CONTAINER320 | 24 | "CONTAINER 320" |
| SRDD_REPORT300_CONTAINER320_CONTAINER330 | 22 | "CONTAINER 330" |
| SRDD_REPORT300_CONTAINER320_MULTISELECT340 | 36 | "MULTISELECT 340" |

Similarly, associated rows added to the table cross-reference table 240 are shown in Table 13.

**Table 13**

| SRDD_TABLE_XREF | | | | |
|---|---|---|---|---|
| ABLE_ID | TABLE_NAME | TABLE_MEANING | PRIMARY_KEY | PARENT_ TABLE_ID |
| | SRDD_GR1 | GENERIC REPORT 1 | iGR1_ID | 0 |
| | SRDD_GR1_CON TAINER_1 | CONTAINER 1 | iGR1_CONT AINER_1_ID | 1 |
| | SRDD_GR1_CON TAINER_2 | CONTAINER 2 | iGR1_CONT AINER_2_ID | 1 |
| | SRDD_GR1_CONTAINER_2_CONTAINE R_3 | CONTAINER 3 | iGR1_CONTAINER_2 _CONTAINER_3_ID | 3 |
| | SRDD_GR1_CON TAINER_2_MULTISEL ECT_1 | MULTISELECT 1 | iGR1_CONT AINER_2 _MULTISELECT_1_ID | 3 |

In some embodiments, the first instance of a particular structured report received with the report data manager 210 consumes a relatively large amount of database space for creation of all associated tables, indexes, constraints, and triggers in the report data database 212. A default size of the report data database 212 can be 1 gigabyte. The size of the report data database 212 can also be altered through database utilities, such as the Microsoft® SQL Server Enterprise Manager® utility available on most versions of Microsoft® SQL Server software.

A structured report is delivered to the report data manager 210 as a text string (e.g., an XML text string) stored in a "report" attribute of a "completion" message. In some embodiments, a "completion" message includes an "approved" message and/or a "transcribed" message. The report data manager 210 parses the text stream into a list of report concepts. In some embodiments, the report data manager 210 parses the structured report by examining the information included in the structured report. For example, the report data manager can use tags and/or other identifiers, such as XML tags, to parse the data included in a structured report. The report data manager can also use a template, such as the structured report template used to create the structured report, in order to identify and parse the data included in a received structured report.

After parsing the structured report into a list of one or more report concepts, the report data manager 210 traverses the report concept list and generates one or more report concept structures that are used to store report concepts to the report data database 212 as report elements 211. In some embodiments, one or more report concept structures are generated for a structured report before a record is written to the report data database 212 since dependencies and/or relationships can exist in a structured report. In order to maintain a level of data integrity, report elements 211 are stored to and/or updated in the report data database 212 for a particular structured report within one transaction. For this reason, the report data manager 210 or the report data database 212 can be configured to prohibit write access to the report data database 212 while the report data manager 210 parses the text string.

A report concept structure generated from a structured report text string with the report data manager 210 contains information used to store a report concept included in the structured report to the report data database 212 as a report element 211. In some embodiments, a report concept structure includes a parameter name and/or identifier attribute (e.g., "param_name"). The parameter name attribute can include a unique identifier generated from the coding scheme, the coding scheme version, and the code value of a report concept. In some embodiments, a report concept structure can also include a units label attribute (e.g., "units_label") that specifies a printable units label such as "mm", "mm Hg", etc. A report concept structure can also include a units description attribute (e.g., "units_description") that provides an explanation of the printable units label, such as "millimeters." In addition, a report concept structure can include an element value attribute (e.g., "element_value") that specifies a value of a report concept and an element meaning attribute (e.g., "element_meaning") that specifies a description of a value of a report concept, such as "PATIENT NAME."

In some embodiments, the report data manager 210 uses a parameter class (e.g., "sr_dd_db_param") that encapsulates the above report concept attributes for sorting and database interface and insertion purposes. The parameter class can include database column attributes necessary to map report concepts to physical table columns where the report concepts are stored as report elements 211.

The parameter class includes a group identifier index (e.g., "sr_param_group_id") that places report concepts into parameter groups where report concepts grouped in the same parameter group are stored in the same physical database table. In some embodiments, parameter objects with the same group index are also stored in the same database row of the same database table. The parameter class can also include a table identifier index (e.g., "sr_table_id") that matches a parameter group to a database table object, which is defined in the next section. In some embodiments, the report data manager 210 generates a sorted list of parameter objects that orders report concepts by group identifier and groups report concepts that are to be stored in the same database table, starting at a top-level report table.

The report data manager 210 uses a table class (e.g., "sr_dd_table") that groups bookkeeping items for each report, container, or multi-select concept that requires a database table. The table class includes a table identifier attribute (e.g., "sr_table_id") that, as described above, identifies a database table object. The table class can also include a table name attribute (e.g., "table_name") that specifies a name of a table of the report data database 212, a table meaning attribute (e.g., "table_meaning") that specifies a meaning or description of a table of the report data database 212, and/or a last identifier attribute (e.g., "dd_last_id") that, if assigned, contains a primary key value of the last record stored in a database table. The table class can further include a report identifier attribute (e.g., "report_uid"), a study instance identifier attribute (e.g., "study_uid"), and a patient identifier attribute (e.g., "patient_uid"). In addition, the table class can include a parent table identifier attribute (e.g., "sr_parent_table_id") that specifies a table identifier attribute for a parent table of a particular database table.

The table class encapsulates the above attributes of a report concept for sorting and retrieval. In some embodiments, instances of the table class are placed in a list as new table-producing report concepts (e.g., containers, multi-select report concepts, etc.) are encountered in the report concept list generated when the report data manager 210 parses the structured report. Initially, a list of table objects or instances can be small and can contain only a few tables. Sorting, therefore, may not be necessary for data retrieval.

The report data manager 210 uses additional classes to parse and/or decompose structured reports into report concept structures and store corresponding report elements 211 to the report data database 212. For example, the report data manager 210 can use a column information class to hold database table column information necessary to compare a current database state to an incoming report structure as described below. Since column statistics can be directly related to physical tables, column information objects can be placed in a list within the table objects.

The report data manager 210 can also use a parameter manager class to maintain lists of table objects and/or parameter objects for sorting, querying, and data packaging. The parameter manager class includes a main data store for an entire report structure and its data and units. The report data manager can further use a text string manager class that provides a static class that parses a structured report, identifies report concepts and related report concept structures, and populates a parameter manager object with table and/or column data.

In addition, the report data manager 210 uses a utilities class as a static class that provides a mechanism for converting parameters names to database object names.

In some embodiments, the report data database manager 210 uses an existence manager class as a static class configured to query the report data database 212 for current table structures (e.g., columns and/or column types) and to query for a possible existence of a report instance in the report data database 212. Objects of the existence manager class can return an instance of the parameter manager class that includes existence flags set for each table and/or column that indicates possible table maintenance as described below.

The report data manager 210 can also use a statement class in order to build an ordered list of commands for one parameter group of parameter objects for table maintenance, data record insert, and/or data record update. Furthermore, the report data manager 210 can use a statement builder class to build an ordered list of commands for a structured report. The list of commands can include table maintenance commands, data record insert commands, and/or data record update commands.

In some embodiments, the report data manager 210 can use a message handler class that inherits functionality from another message handler class. For example, the message handler class used with the report data manager 210 can inherit from a message handler class used with the SR application and/or the entire medical system 20 that manages attribute/value pairs protocol with sequenced items messages (e.g., MCF messages). The message handler class used with the report data manager 210 includes a "handle_message" method that obtains "completion" messages, parses a structured report included in the message (e.g., using the text string manager class), determines a current database state (e.g., using the existence manager class), creates an ordered list of commands (e.g., using the statement builder class), and executes each command within a database transaction block.

Configuration file attributes for the report data manager 210, which can be modified in order to modify the configuration of the report data manager 210 and, ultimately, the functionality and behavior of the report data manager 210, are accessible through a configuration manager class. Component configuration file attributes are stored in a component configuration file that includes settings for the report data manager, such as log settings. For example, the component configuration file can include a log level setting that specifies what type of information should be logged (e.g., errors only, errors and performance data, etc.) and/or the location of a corresponding log file.

In addition to the above classes, the report data manager 210 can use other classes, files, functions, stored procedures, and/or applications in order to parse structured reports and store report elements 211extracted from a structured report to the report data database 212. For example, the report data manager 210 can use a batch file to drop and create the report data database 212. In some embodiments, the batch file is automatically executed as part of a report data database 212 and/or report data manager 210 install sequence. The batch file can also create maintenance tables, stored procedures, and insert seed index records.

In some embodiments, the batch file accesses one or more script files. The batch file accesses a drop database script file that drops the report data database 212. The drop database script file removes a user and/or a login for the report data database 212. The batch file accesses a build database script file that creates the report data database 212. The build database script file can also add a user and/or login for the report data database 212, if necessary.

In addition, the batch file can access a setup database script file that creates one or more maintenance tables for the report data database 212. The setup database script file can also insert seed index records. In addition, the setup database script file can create one or more stored procedures. For example, the setup database script file can create a retrieve next identifier procedure that determines a next available identifier value for a given identifier name. In some embodiments, the results of executing the stored retrieve next identifier procedure are accessed via an output parameter. The setup database script file can also create a get next identifier procedure that also determines a next available identifier value for a given identifier name. The results of executing the get next identifier procedure, however, can be accessed via a result set.

In some embodiments, the setup database script file also creates a get basic column information procedure that returns a result set containing a list of current column names and datatypes in a given table. The setup database script file creates an insert table cross-reference procedure that inserts a record into the table cross-reference table 240. The insert table cross-reference table procedure also normalizes parent table names to table identifier cross-reference values.

In addition, the setup database script file can create a lookup table procedure and/or a get report table procedure. The lookup table procedure looks for the presence of a current report record associated with a given table name, and the get report table procedure returns a unique top-level table name for a distinct report template.

The following report labeled Report 1 is an exemplary "ECHOCARDIOGRAM MEASUREMENTS" structured report and illustrates how the above classes can organize report concepts included in a structured report for storage in the report data database 212 as described in the following examples.

### Report 1

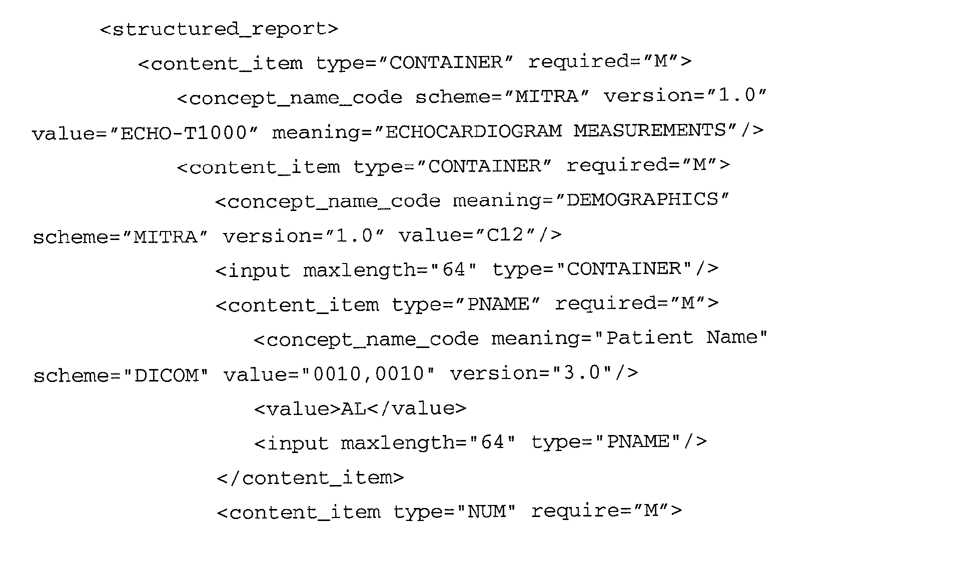

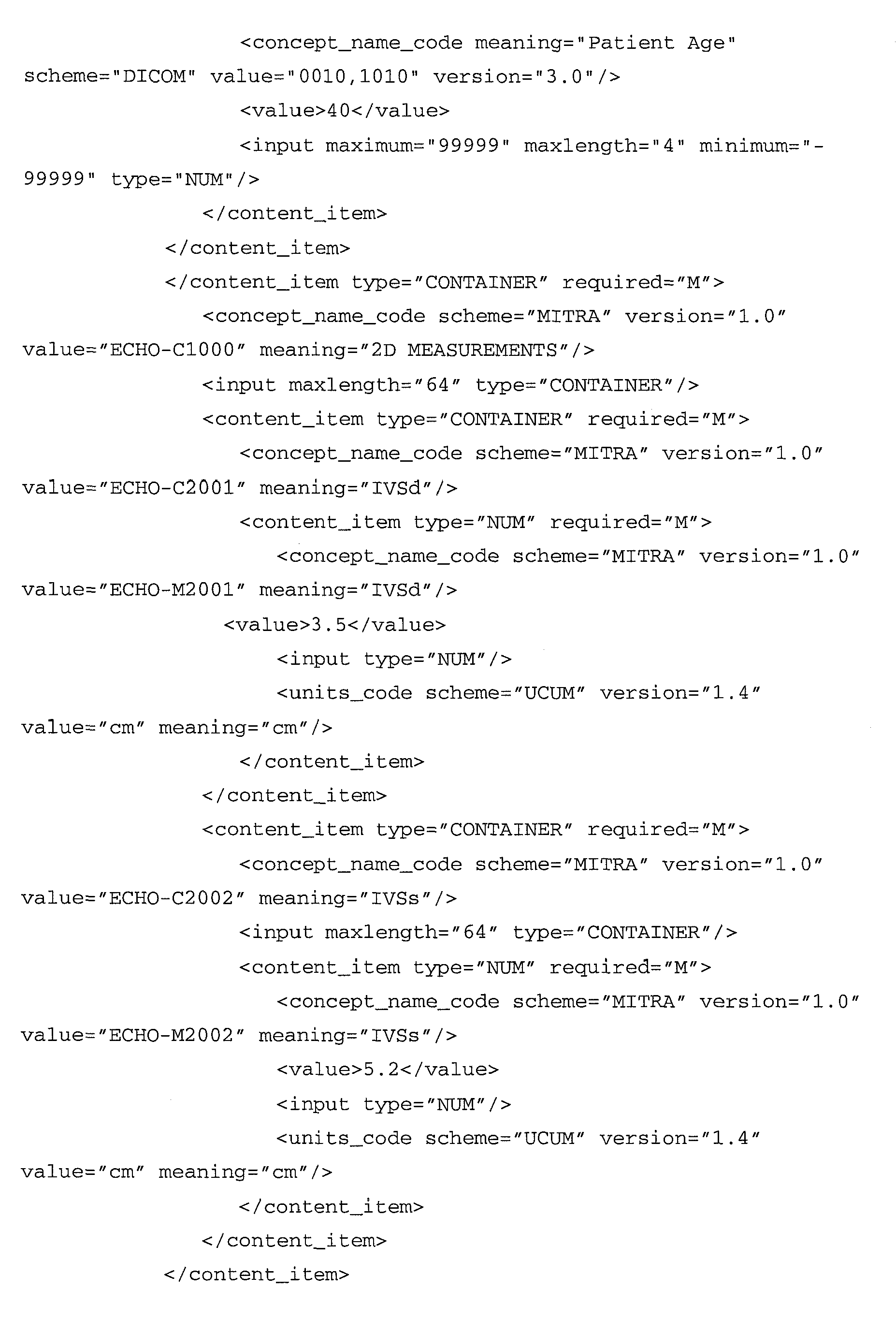

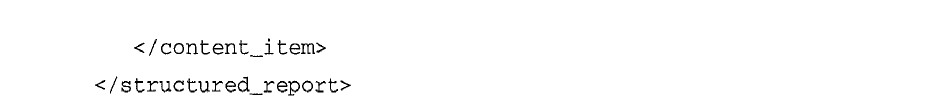

Report 1 represents a flat report that does not include any multicontainer concepts or multi-select report concepts. Therefore, the report data manager 210 establishes and accesses one table defined for Report 1. An exemplary structure of a table object representing the database table associated with Report 1 is illustrated in Table 16 below.

**Table 16**

| Sr_dd_table attribute | Value |
|---|---|
| Sr_table_id | 1 |
| table_name | SRDD_EM |
| table_meaning | ECHOCARDIOGRAM MEASUREMENTS |
| sr_parent_group_id | 0 (top-level) |
| dd_last_id | 0 |
| report_uid | 0 (contains the message report_uid) |
| study_uid | 0 (contains the message study_uid) |
| patient_uid | 0 (contains the message patient_uid) |

Although Report 1 includes eight report concepts in the top-level report table (i.e., a "DEMOGRAPHICS" container, a "Patient Name" concept, a "Patient Age" concept, a "2D MEASUREMENTS" container, a "IVSd" container, a "IVSd" concept, a "IVSs" container, and a "IVSs" concept), four of the eight report concepts are single container elements (i.e., the "DEMOGRAPHICS" container, the "2D MEASUREMENTS" container, the "IVSd" container," and the "IVSs" container).

In some embodiments, single container elements affect only the column name of the concepts contained within (e.g., column name prefix) and do not produce a report element 211 to add and/or update in the report data database 212. Therefore, the above structured report includes four report concepts to be stored and/or updated as report elements 211 in the report data database 212 in a table associated with the report, such as the table represented by the table object illustrated in Table 16 (i.e., "SRDD_EM.")

Tables 17 and 18 illustrated below represent parameter objects created for the "Patient Name" report concept and the "Patient Age" report concept of Report 1. Since the "Patient Name" report concept and the "Patient Age" report concept are members of the "DEMOGRAPHICS" container, the "DEMOGRAPHICS" unique identifier prefix is added to the identifier for the "Patient Name" column and the "Patient Age" column.

**Table 17**

| Sr_dd_db_param attribute | value |
|---|---|
| Sr_param_group_id | 1 |
| Sr_table_id | 1 |
| Sr_param_name | DEMOGRAPHICS_PATIENT_NAME |
| code_meaning | PATIENT NAME |
| element_value | AL |
| element_type | PNAME |
| units_label | NULL |
| units_meaning | NULL |

**Table 18**

| sr_dd_db_param attribute | value |
|---|---|
| Sr_param_group_id | 1 |
| Sr_table_id | 1 |
| Sr_param_name | DEMOGRAPHICS_PATlENT_AGE |
| code_meaning | PATIENT AGE |
| element_value | 40 |
| element_type | NUM |
| units_label | NULL |
| units_meaning | NULL |

The "IVSd" container of Report 1 is a member of the "2D Measurements" container and includes an "IVSd" report concept. In order to guarantee a unique column identifier, unique identifiers of both parent containers are used to generate the prefix for the "IVSd" concept. The prefix name can be generated in container level order starting with the top-level container (i.e, "2D Measurements"). Tables 19 and 20 illustrate parameter objects generated with the report data manager for the "IVSd" report concept and the similarly structured "IVSs" report concept of Report 1.

**Table 19**

| Sr_dd_db_param attribute | Value |
|---|---|
| Sr_param_group_id | 1 |
| sr_table_id | 1 |
| sr_param_name | 2D_MEASUREMENTS_IVSD_IVSD |
| code_meaning | IVSd |
| element_value | 3.5 |
| element_type | NUM |
| units_label | Cm |
| units_meaning | Cm |

**Table 20**

| sr_dd_db_param attribute | Value |
|---|---|
| sr_param_group_id | 1 |
| sr_table_id | 1 |
| sr_param_name | 2D_MEASUREMENTS_IVSS_IVSS |
| code_meaning | IVSs |
| element_value | 5.2 |
| element_type | NUM |
| units_label | Cm |
| units_meaning | Cm |

Using the table object and parameter objects generated with the report data manager 210 after parsing Report 1 (as shown in Tables 16 - 20), the report data manager 210 knows what tables are needed in the report data database 212 in order to store the report elements included in Report 1. In particular, the report data manager 210 knows that a database table with the name "SRDD_EM" is needed in the report data database 212. The report data manager 210 also knows that the required table needs to contain at least the columns with the following names and datatypes as set up in Table 21, which provides an exemplary structure of the required table.

**Table 21**

| SRDD_EM | |
|---|---|
| Column | datatype |
| iEM_ID | int |
| vchREPORT_UID | varchar(128) |
| vchSTUDY_UID | varchar(128) |
| vchPATIENT_UID | varchar(128) |
| dtINSERTED_DT | datetime |
| dtUPDATED_DT | datetime |
| vchDEMOGRAPHICS_PATIENT_NAME | varchar(64) |
| fDEMOGRAPHICS_PATIENT_AGE | float |
| f2D_MEASUREMENTS_IVSD_IVSD | float |
| unit2D_MEASUREMENTS_IVSD_IVSD | varchar(24) |
| f2D_MEASUREMENTS_IVSS_IVSS | float |
| unit2D_MEASUREMENTS_IVSS_IVSS | varchar(24) |

If the "2D MEASUREMENTS" container of Report 1 is changed to a multicontainer, the "IVSd" and "IVSs" report concepts, which are members of the "IVSd" and "IVSs" containers, respectively, become members of a new parameter group identifier corresponding to a specific table created for the "2D MEASUREMENTS" multicontainer. Table 22 illustrates an exemplary table object generated with the report data manager 210 for the "2D MEASUREMENTS" multicontainer, and Tables 23 and 24 respectively illustrate exemplary parameter objects generated with the report data manager 210 for the "IVSd" and "IVSs" report concepts.

**Table 22**

| Sr_dd_table attribute | Value |
|---|---|
| sr_table_id | 2 |
| table_name | SRDD_2D_MEASUREMENTS |
| table_meaning | 2D MEASUREMENTS |
| sr_parent_table_id | 1 (ECHOCARDIOGRAM MEASUREMENTS) |
| dd_last_id | 0 |
| study_uid | |
| report_uid | |

**Table 23**

| sr_dd_db_param attribute | Value |
|---|---|
| sr_param_group_id | 2 |
| sr_table_id | 2 |
| sr_param_name | IVSd_IVSd |
| code_meaning | IVSd |
| element_value | 3.5 |
| element_type | NUM |
| units_label | cm |
| units_meaning | cm |

**Table 24**

| sr_dd_db_param attribute | value |
|---|---|
| sr_param_group_id | 2 |
| sr_table_id | 2 |
| sr_param_name | IVSs_IVSs |
| code_meaning | IVSs |
| element_value | 5.2 |
| element_type | NUM |
| units_label | cm |
| units_meaning | cm |

With Report 1 altered to include a "2D MEASUREMENTS" multicontainer, the report data manager 210 can expect two tables (i.e., "SRDD_EM" and "SRDD_EM_2D_MEASUREMENTS") in the report data database 212. Tables 25 and 26 illustrate exemplary structures (e.g., columns and datatypes) for the two expected tables. It should be understood that corresponding units columns for report element tables are not illustrated in the following tables for brevity, but can be included in order to provide meaningful query results.

**Table 25**

| SRDD_EM | |
|---|---|
| Column | datatype |
| iEM_ID | int |
| vchREPORT_UID | varchar(128) |
| vchSTUDY_UID | varchar(128) |
| vchPATIENT_UID | varchar(128) |
| dtINSERTED_DT | datetime |
| dtUPDATED_DT | datetime |
| vchDEMOGRAPHICS_PATIENT_NAME | varchar(64) |
| fDEMOGRAPHICS_PATIENT_AGE | float |

**Table 26**

| SRDD_EM_2D_MEASUREMENTS | |
|---|---|
| Column | datatype |
| iEM_2D_MEASUREMENTS | int |
| iEM_ID | int |
| fIVSD_IVSD | float |
| unitIVSD_IVSD | varchar(24) |
| fIVSS_IVSS | float |
| unitIVSS_IVSS | varchar(24) |

As described above with respect to Fig. 17, a report concept of type "Code" can have an input type of "SELECT" or "MULTISELECT." A "SELECT" input type allows one selection of a value for the report concept from a list of options. Report concepts of type "code" with an input type of "SELECT" are treated as single concepts and can be stored as a column in a table in the report data database 212. On the other hand, a report concept of type "Code" with an input type of "MULTISELECT" allows multiple selections from a list of options and can require a separate database table. The separate database table for the multi-select report concept contains one primary key index column and one value column.

Report 2 (illustrated below) represents Report 1 altered to include two report concepts of type "Code.

### Report 2

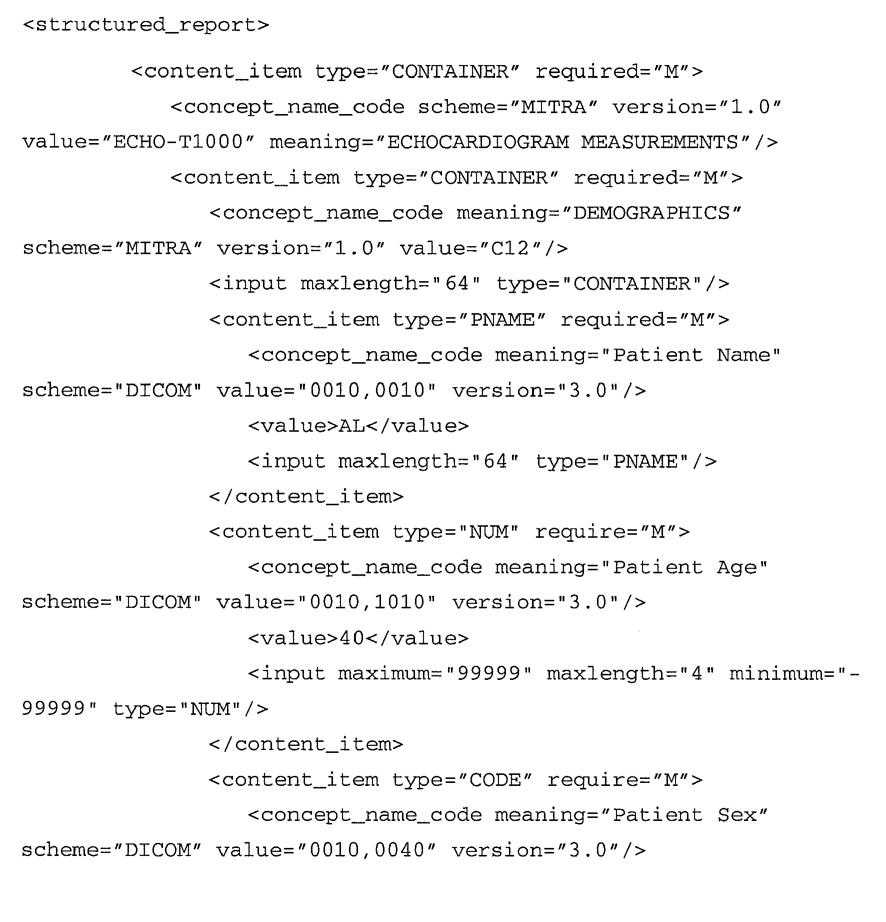

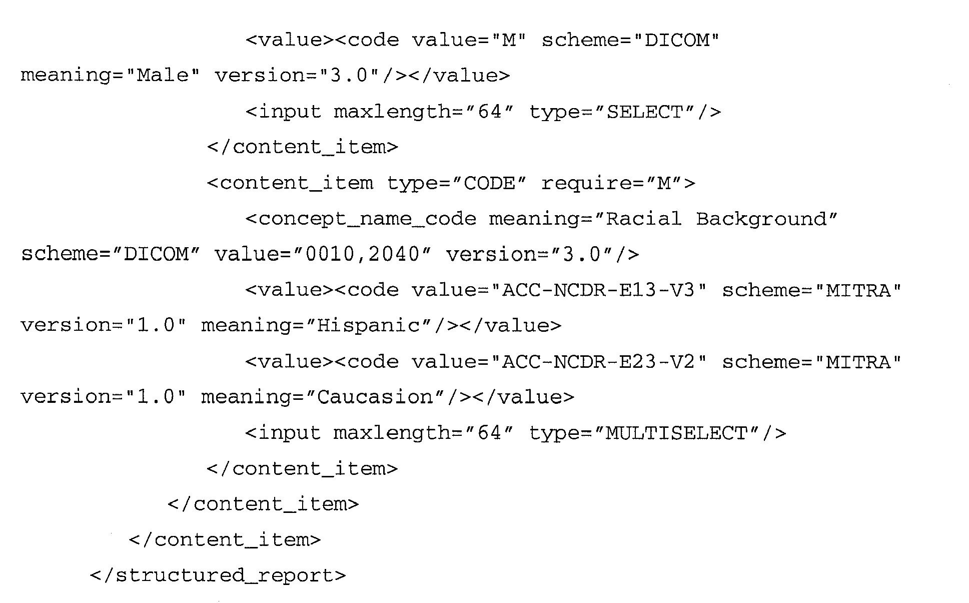

As in the previous examples for Report 1, the top-level report container of Report 2 causes the report data manager 210 to generate a table object for the top-level report container (i.e., the "ECHOCARDIOGRAM MEASUREMENTS" container). Table 27 illustrates an exemplary table object generated for the top-level report container of Report 2.

**Table 27**

| Sr_dd_table attribute | Value |
|---|---|
| Sr_table_id | 1 |
| table_name | SRDD_EM |
| Table_meaning | ECHOCARDIOGRAM MEASUREMENTS |
| sr_parent_group_id | 0 (top-level) |
| dd_last_id | 0 |
| report_uid | 0 (contains the message report_uid) |
| Study_uid | 0 (contains the message study_uid) |

As described in the previous examples for Report 1, the "Patient Name" report concept and the "Patient Age" report concept of Report 2 are members of the "DEMOGRAPHICS" container. Therefore, the "DEMOGRAPHICS" unique identifier prefix can be added to the "Patient Name" report concept column identifier and the "Patient Age" report concept column identifier. Tables 28 and 29 illustrates exemplary parameter objects generated with the report data manager 210 for the "Patient Name" report concept and the "Patient Age" report concept, respectively.

**Table 28**

| sr_dd_db_param attribute | Value |
|---|---|
| sr_param_group_id | 1 |
| sr_table_id | 1 |
| sr_param_name | DEMOGRAPHICS_PATIENT_NAME |
| code_meaning | PATIENT NAME |
| element_value | AL |
| element_type | PNAME |
| Units_label | NULL |
| Units_meaning | NULL |

**Table 29**

| sr_dd_db_param attribute | Value |
|---|---|
| sr_param_group_id | 1 |
| sr_table_id | 1 |
| sr_param_name | DEMOGRAPHICS_PATIENT_AGE |
| code_meaning | PATIENT AGE |
| element_value | 40 |
| element_type | NUM |
| Units_label | NULL |
| Units_meaning | NULL |

Report 2 includes a "Patient Sex" report concept of type "Code" and an input type of "SELECT," which specifies that only one value selection is allowed for the report concept. As described above, since the report concept has an input type of "SELECT," the report concept can map to a column in the table corresponding to the top-level report container. The "Patient Name" report concept, the "Patient Age" report concept, and the "Patient Sex" report concept all map to columns in the table object for the top-level report container. Therefore, parameter objects representing these three concepts have the same parameter group identifier attribute value and the same table identifier attribute value. Table 30 illustrates an exemplary parameter object representing the "Patient Sex" report concept.

**Table 30**

| Sr_dd_db_param attribute | Value |
|---|---|
| Sr_param_group_id | 1 |
| sr_table_id | 1 |
| sr_param_name | DEMOGRAPHICS_PATIENT_SEX |
| code_meaning | PATIENT SEX |
| element_value | Male |
| element_type | CODE |
| units_label | NULL |
| units_meaning | NULL |

As also shown above, Report 2 includes a "Patient Racial Background" report concept of type "Code" and an input type of "MULTISELECT," which specifies that multiple values can be selected for the report concept. As described above, separate container tables can be generated for report concepts having an input type of "MULTISELECT." Table 31 illustrates an exemplary table object representing the "Patient Racial Background" report concept of Report 2.

**Table 31**

| Sr_dd_table attribute | Value |
|---|---|
| sr_table_id | 2 |
| Table_name | SRDD_EM_RACIAL_BACKGROUND |
| Table_meaning | Racial Background |
| sr_parent_group_id | 1 (ECHOCARDIOGRAM MEASUREMENTS) |
| dd_last_id | 0 |
| report_uid | 0 (contains the message report_uid) |
| Study_uid | 0 (contains the message study_uid) |

The two racial background selections included in Report 2 are two parameter records with distinct parameter group identifier attributes that each reference the same parent container table (i.e., the container table generated for the "MULTISELECT" report concept). Table 32 illustrates an exemplary parameter object representing the first racial background selection, and Table 33 illustrates an exemplary parameter object representing the second racial background selection.

**Table 32**

| sr_dd_db_param attribute | Value |
|---|---|
| sr_param_group_id | 2 |
| sr_table_id | 2 |
| sr_param_name | RACIAL_BACKGROUND |
| code_meaning | Racial Background |
| container_id | DEMOGRAPHICS |
| element_value | Hispanic |
| element_type | CODE |
| units_label | NULL |
| units_meaning | NULL |

**Table 33**

| sr_dd_db_param attribute | Value |
|---|---|
| sr_param_group_id | 3 |
| sr_table_id | 2 |
| sr_param_name | RACIAL_BACKGROUND |
| code_meaning | Racial Background |
| container_id | DEMOGRAPHICS |
| element_value | Caucasion |
| element_type | CODE |
| units_label | NULL |
| units_meaning | NULL |

To summarize the example, Report 2 includes two report concepts of type "Code" that access two distinct database tables. A first report table, "SRDD_EM," holds the top-level report elements 211, a primary key index, and a report, study, and/or patient identifiers (e.g., the "Patient Name" report concept, the "Patient Age" report concept, and the "Patient Sex" report concept). A second container table, "SRDD_EM_RACIAL_BACKGROUND," stores the multiple selections for the "Patient Racial Background" report concept. Tables 34 and 35 respectively illustrate exemplary table structures for the first report table and the second container table.

**Table 34**

| SRDD_EM | |
|---|---|
| Column | Datatype |
| iEM_ID | Int |
| vchREPORT_UID | varchar(128) |
| vchSTUDY_UID | varchar(128) |
| vchPATIENT_UID | varchar(128) |
| dtINSERTED_DT | datetime |
| dtUPDATED_DT | datetime |
| vchDEMOGRAPHICS_PATIENT_NAME | varchar(64) |
| fDEMOGRAPHICS_PATIENT_AGE | float |
| vchDEMOGRAPHICS_PATIENT_SEX | varchar(128) |

**Table 35**

| SRDD_EM_RACIAL_BACKGROUND | |
|---|---|
| column | datatype |
| iEM_ID | int |
| iEM_RACIAL_BACKGROUND_ID | int |
| vchCONTAINER | varchar(128) (i.e. DEMOGRAPHICS) |
| vchRACIAL_BACKGROUND | varchar(128) |

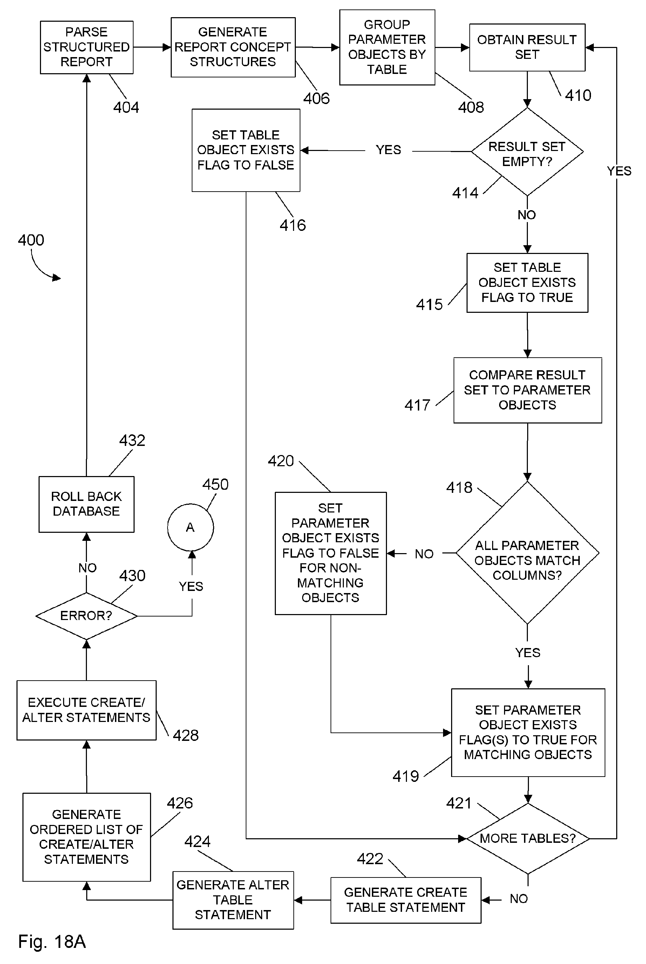

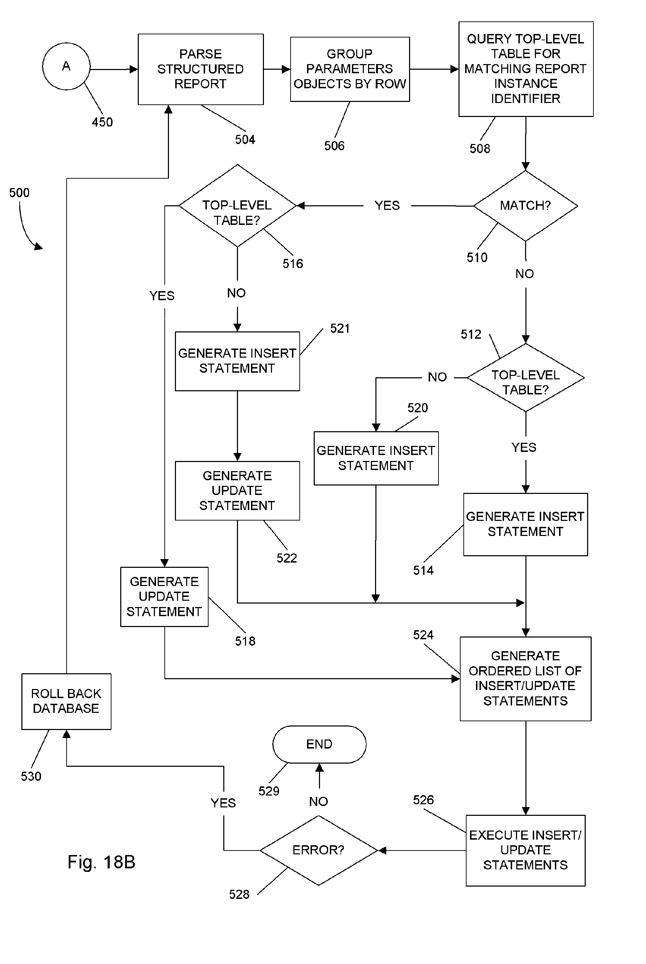

In some embodiments, once the report data manager 210 consumes a valid structured report message, the structured report and any corresponding report data database 212 updates and/or deletions can be processed in two passes or modes, as shown in Figs. 18A and 18B.

In a first or "maintenance mode" 400 (illustrated in Fig. 18A), the report data manager 210 parses the structured report (step 404) as described above in order to generate one or more report concept structures (step 406). The report data manager 210 then organizes parameter objects into parameter groups corresponding one-to-one with physical database tables (step 408). To do so, the report data manager 210 requests a current database state (also referred to as a result set) that lists available columns for each table object from the report data database 212 (step 410) and compares the result set to a list of generated parameter objects (step 412).

In some embodiments, the report data manager 210 uses one or more existence flags in order to track the results of comparing the result set to the list of generated parameter objects. The report data manager 210 uses a table object existence flag, and, if the result set contains at least one row (step 414), the report data manager 210 sets the table object existence flag to true (step 415). Otherwise, the report data manager 210 sets the table object existence flag to false (step 416).

The report data manager 210 also uses one or more parameter object existence flags. For each result set, the report data manager 210 compares a column of a particular table, to the parameter objects associated with the same table (step 417). If all the parameter objects associated with the table match columns already included in the table (step 418), the report data manager 210 sets a parameter object existence flag associated with each parameter object to true (step 419). If one or more parameter objects do not match a column already included in the table (meaning that the parameter objects represent a new column that should be added to the table), the report data manager 210 sets the parameter object existence flags associated with the one or more non-matching parameter objects to false (step 420). The report data manager 210 also sets the parameter object existence flags associated with any matching parameter objects to true (step 419). The report data manager 210 continues retrieving result sets (step 410), checking for empty result sets (step 414), and comparing the result set to the parameter objects associated with the same table (step 417) as long as additional tables remain to be checked (step 421).

Once all existence flags (i.e., table object existence flags and parameter object existence flag(s)) are set, the report data manager 210 examines each parameter group in order to determine if any database maintenance statements are needed to alter the tables of the report data database 212 and store the report concepts included in the structured report. As mentioned earlier, each parameter group corresponds one-to-one with a table object.

If a table object does not exist, (i.e., a table existence flag is set to false) the report data manager 210 generates a table maintenance statement that includes a create table statement (step 422). The create table statement defines a table object containing all associated parameter objects as columns. The report data manager 210 can also add a primary key constraint on the "id" column of the created table. In addition, the report data manager 210 can add records to the last identifier maintenance table 230 and the table cross-reference maintenance table 240 for use in data manipulation and queries.

In some embodiments, if a top-level table is created, the report data manager 210 creates additional database objects. The report data manager 210 creates a delete trigger object that deletes first-level children table records that reference an affected top-level report record. The report data manager 210 also creates an update trigger that deletes the first-level children table records that reference an affected top-level report record. First-level children table records that reference an affected top-level report record are refreshed or re-inserted after being deleted with the update trigger. In addition, the report data manager 210 can create additional columns, such as columns that uniquely identify a patient examination and results ("vchREPORT_UID"), a study ("vchSTUDY_UID"), and/or a patient ("vchPATIENT_UID") to be added to the top-level table. In some embodiments, the report data manager 210 also creates a non-clustered index on a column that uniquely identifies a report instance in order to help with record query performance and a non-clustered index on a column that tracks update dates and/or times or creation dates and/or times in order to help with archival and cleanup performance. As described above, the report data manager 210 can also add records to the report cross-reference table 250.

If, on the other had, a created table is a child table, the report data manager 210 creates and adds primary keys for all parent tables chaining-up to the top-level table as columns of the created child table. The report data manager may also place foreign key constraints on the parent table key columns with a cascading delete. In addition, the report data manager 210 can add a column to the created child table in order to store a possible parent single-container identifier for the child multicontainer table or multi-select table.

If a table object exists, the report data manager 210 examines the corresponding parameter object existence flags. If at least one parameter object existence flag is set to false, the report data manager 210 generates a table maintenance statement that includes an alter table statement (step 424). The alter table statement adds columns to an existing table associated with parameter objects whose parameter object existence flags are set to false. The report data manager 210 also sets a recompile switch on the altered table so that any stored procedures accessing the altered table recompile at the next execution.

In some embodiments, the report data manager 210 places table maintenance statements in an ordered statement list beginning with the top-level table and working down through the table tree (step 426). The table maintenance statements are executed in order within a single transaction block (step 428). If any statement execution produces an error (step 430), the whole set of statements is rolled back to a previous database state (step 432). The ability to rollback the statements can be used in order to improve data stability of the report data database 212. In some embodiments, if the statements are rolled back, the report data manager 210 reprocesses the originally obtained structured report in a "maintenance" mode 400 and/or processes a subsequently received structured report in a "maintenance" mode 400 (step 402).

If an error does not occur when executing any create and/or alter statements generated during the maintenance mode, the report data manager 210 can proceed to a second or "unknown" mode 500 as illustrated in Fig. 18B (the connection between the "maintenance" mode 400 and the "unknown" mode is illustrated in Figs. 18A and 18B as connector A (step 450)). During the "unknown" mode 500, the report data manager 210 parses the same structured report (step 504) but organizes the generated parameter objects into groups corresponding to data rows of tables (step 506). Then, the report data manager 210 queries the report data database 212 for an entry in the report data database 212 that matches a report instance identifier included in the structure report and/or the "completion" message (step 508). If a record is found that has a matching report instance identifier (step 510), the report data manager 210 switches to an "UPDATE" mode. Otherwise, the report data manager 210 switches to an "INSERT" mode.

In the "INSERT" mode, if the table being inserted into is a top-level table (step 512), the report data manager 210 generates a table modification statement that includes an insert statement which references specific columns containing non-null report elements 211 of the top-level table (step 514). The insert statement, when executed, adds report elements 211 (obtained from the report concept structures) into the report data database 212. The report data manager 210 can also set insert date and/or time columns or update date and/or time columns to the time of the medical system 20. In some embodiments, the report data manager 210 uses a date retrieval function, such as the SQL function getdate(), in order to obtain the data and/or time of the system 20. The report data manager 210 can also obtain a primary key value from the get next identifier stored procedure, which references the last identifier maintenance table 230 as described above.

In the "UPDATE" mode, if the table being updated is a top-level table (step 516), the report data manager 210 generates a table modification statement that includes an update statement that sets columns containing report elements 211 (step 518). The update statement updates previously stored report elements 211 based on the matching report concept structures. The update statement can also trigger a cascading delete of all child tables in order to force a refresh of rows for a particular report instance. The report data manager 210 can also set update data and/or time columns or update date and/or time columns to the time of the medical system 20 using a date retrieval function. The report data manager 210 can also obtain the primary key value for the record to be updated using the report identifier lookup stored procedure as described above.

If the table being updated or inserted into is not a top-level table (step 512 or step 516), the report data manager 210 generates a table modification statement that includes an insert statement which references specific columns containing report elements 211 to be updated or inserted (step 520 or step 521). The report data manager 210 generates a primary key value for the insert statement from the get next identifier stored procedure that references the last identifier maintenance table 230 as described above.

In some embodiments, generated table modification statements (i.e., insert statements and update statements) are placed in an ordered statement list starting with the top-level report table and working down through a table hierarchical tree (step 524). The statements are then executed in order within a single transaction block (step 526). If no errors occur (step 528), the processing of a structured report is complete (step 529). Any nonrecoverable statement execution errors that occur (step 528) result in a transaction rollback that leaves the report data database 212 in a previous state in order to maintain data integrity (step 530). In some embodiments, if a recoverable error occurs, such as a database deadlock error, the report data manager 210 retires the block of statements being executed with the error occurred a configurable number of times with a configurable time delay between each retry. After the report data manager 210 retries executing the block of statements the configurable number of times, the report data manager 210 can consider the recoverable error nonrecoverable and can perform a transaction rollback. After rolling back the database 212, the report data manager 210 can attempt to re-process the same structured report in an "unknown" mode 500 (step 504).

As described above, once report elements 211 are stored in the report data database 212, the query engine 214 can obtain the report elements 211 for various processing purposes. Several query examples are provided below. Query 1 (below) includes a query of specific items in the top-level table for a given report instance of a report structured as illustrated above in Report 1.

### Query 1

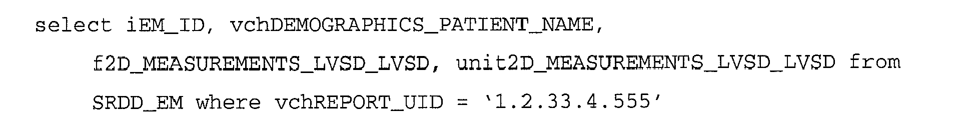

In addition, all child records associated with a specific report can be accessed by selecting records from the child table using the top-level table primary key as the search criteria in a "where" clause. For example, Query 2 illustrates an exemplary query of racial background records for a report structured similar to Report 1.

### Query 2

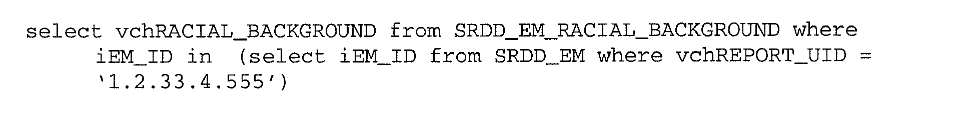

Query 3 illustrates the query illustrated in Query 2 written using a local variable.

### Query 3

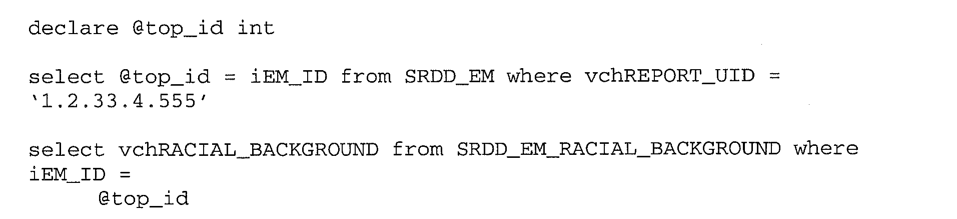

Query 4 illustrates how to query information when only meanings are known for a desired report template. In particular, Query 4 illustrates a query of all columns in the top-level table for a particular report instance of a report structure similar to Report 1.

### Query 4

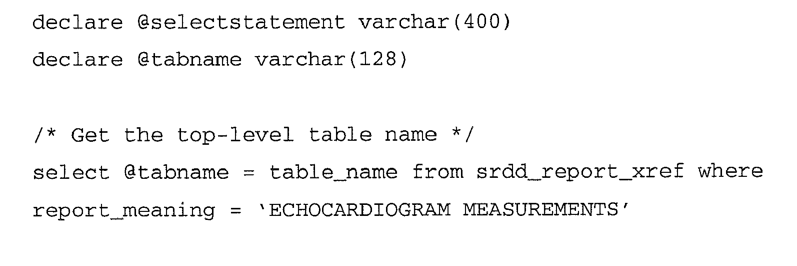

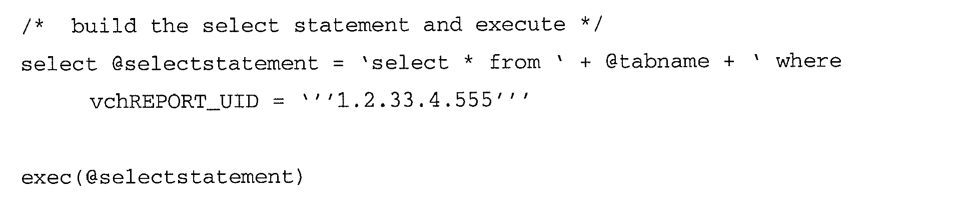

Query 5 illustrates how to query all first-level child tables of a report structured similar to Report 1. Query 5 shows the use of a cursor, but temporary tables and/or other mechanisms can be just as effective.

### Query 5

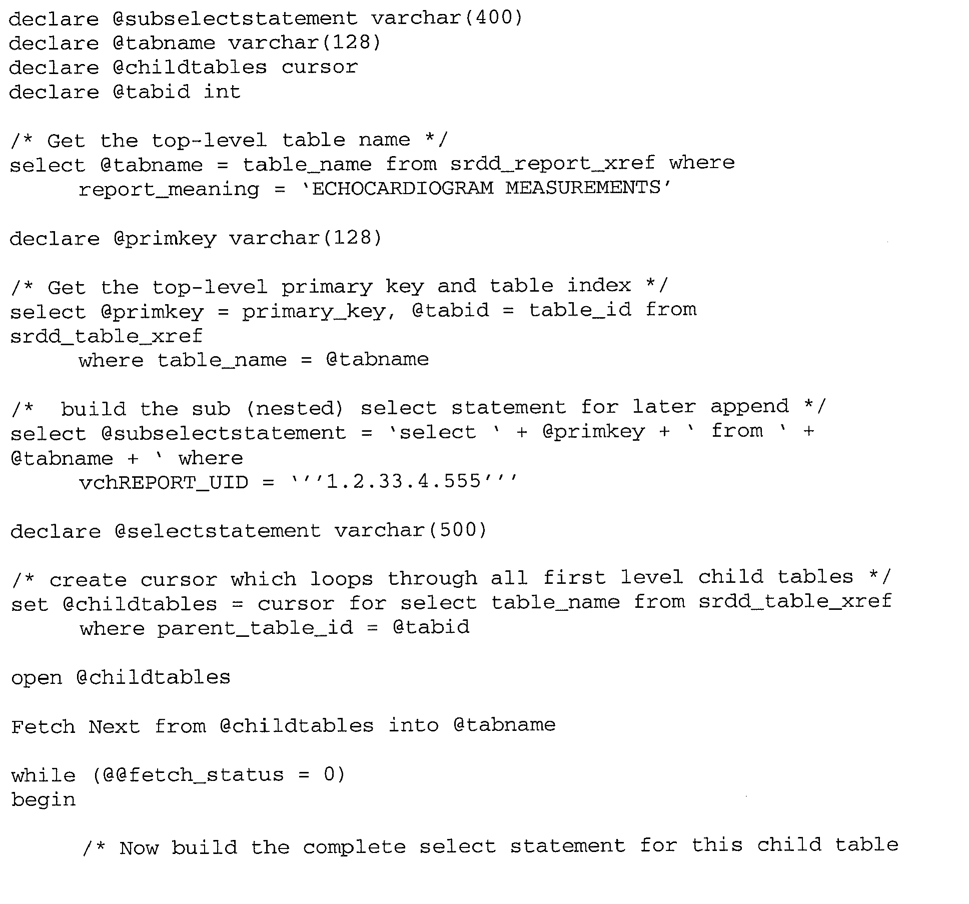

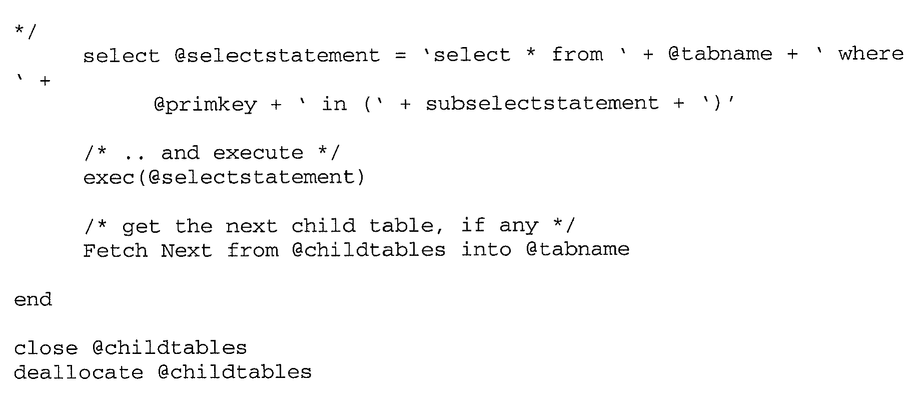

To return all rows in all levels of child tables for a specific report instance, the following query (Query 6) can be executed. As shown in Query 6, a query can use the get table data stored procedure, as described above, in order to return result sets for all tables for a report template meaning and report instance.

### Query 6

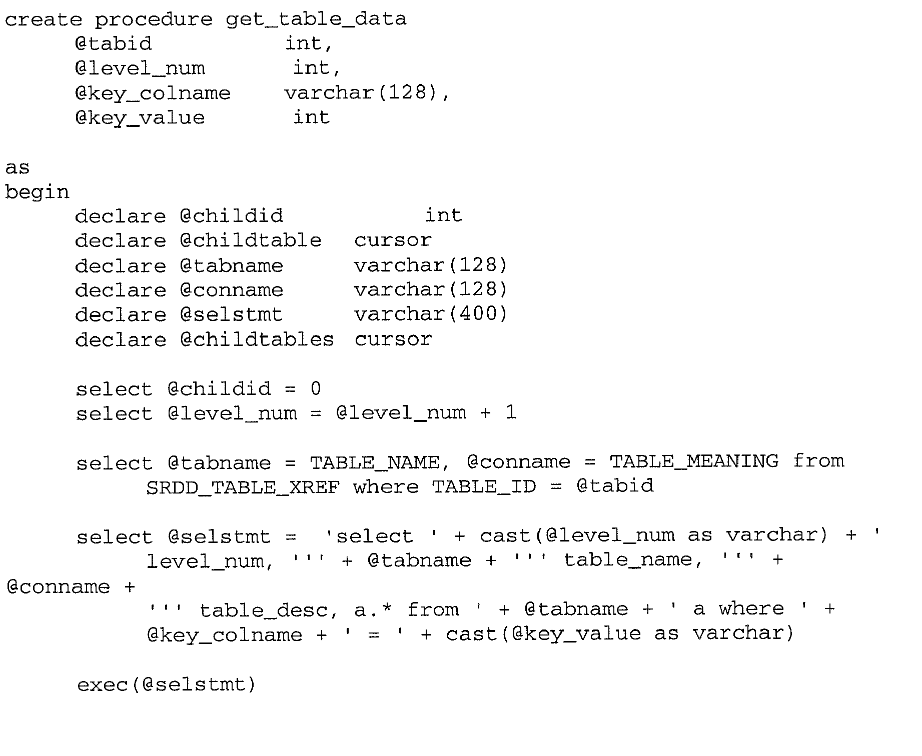

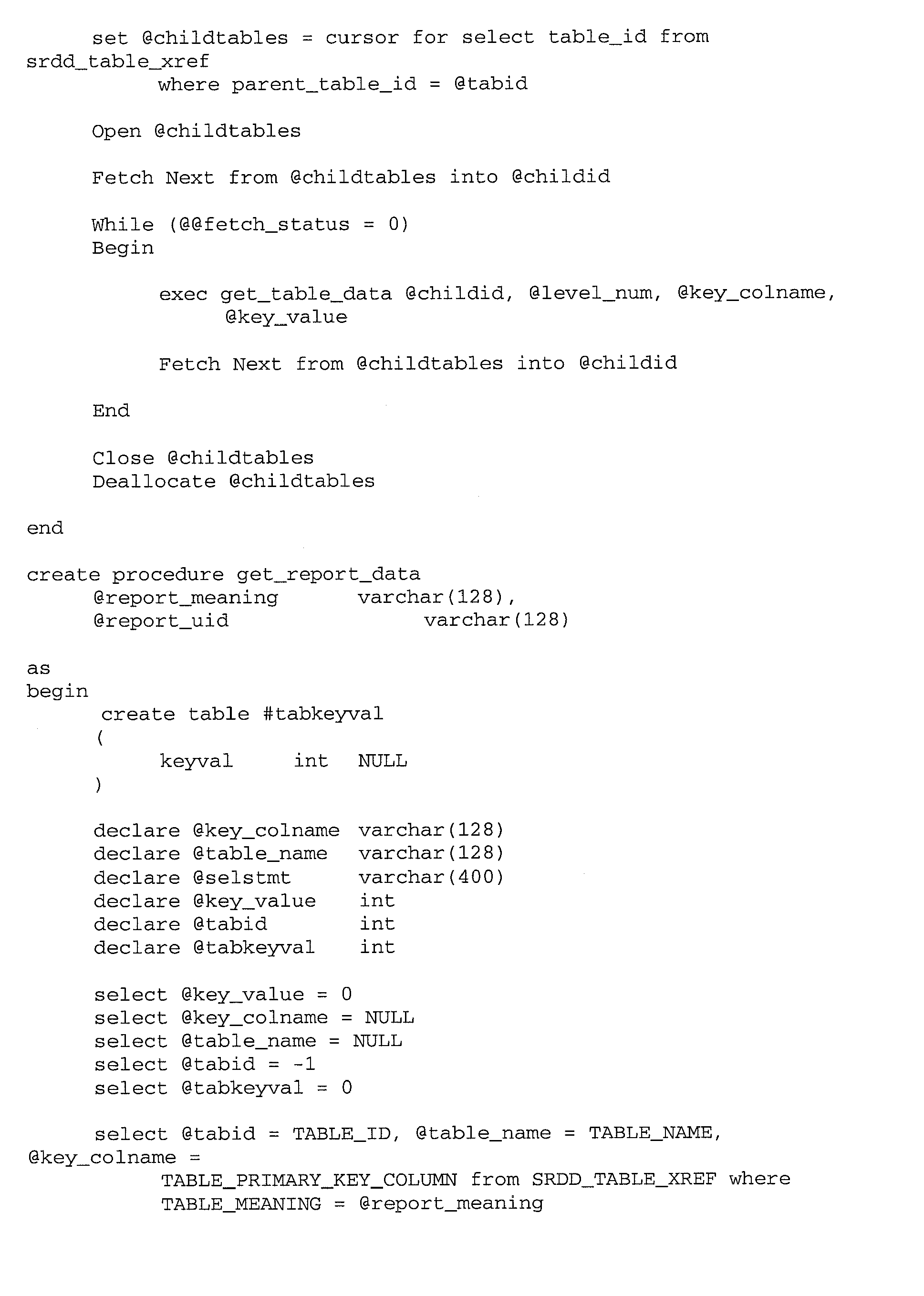

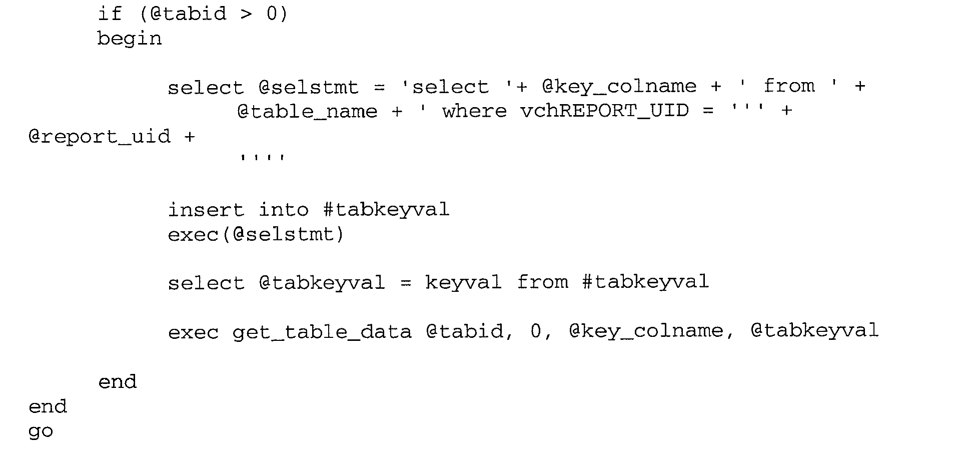

As should also be apparent to one of ordinary skill in the art, the systems shown in the figures are models of what actual systems might be like. As noted, many of the modules and logical structures described are capable of being implemented in software executed by a microprocessor or a similar device or of being implemented in hardware using a variety of components including, for example, application specific integrated circuits ("ASICs"). Terms like "processor" may include or refer to both hardware and/or software. In addition, throughout the specification capitalized terms are used. Such terms are used to conform to common practices and to help correlate the description with the coding examples and drawings. However, no specific meaning is implied or should be inferred simply due to the use of capitalization. Thus, the claims should not be limited to the specific examples provided.

Various features and advantages of the invention are set forth in the following claims.

## Claims

1. A system for processing information and creating structured reports, the system comprising:
- a business logic server;
- a structured object repository configured to hold a plurality of structured report templates, the structured report templates based on a common schema;
- at least one inbound message device configured to receive data from at least one source of data in a message-oriented protocol, to convert the data from the at least one source of data to a format recognized by the business logic server, and to send the converted data to the business logic server;
- a report data manager; and
a report data database, the business logic server configured to obtain at least one structured report template from the plurality of structured report templates and to create a structured report by inserting the converted data into the at least one structured report template, the report data manager configured to obtain the structured report, to determine one or more report elements included in the structured report, and to store the one or more report elements in the report data database.

2. The system of claim 1 further comprising a query engine configured to query the report data database for the one or more report elements.

3. The system of claim 1 wherein the report data database is configured to include at least one of a report table, a container table, and a maintenance table.

4. The system of claim 1 wherein the report data manager is further configured to parse the structured report and generate one or more report concept structures.

5. A method of storing zero or more report elements included in a structured report, the method comprising:
- obtaining a structured report;
- determining zero or more report elements included in the structured report; and
- storing the zero or more report elements in a report data database.

6. The method of claim 5 further comprising parsing the structured report and generating one or more report concept structures.

7. The method of claim 6 further comprising obtaining a current database state from the report data database.

8. A report data manager configured to receive a structured report, to determine one or more report elements included in the structured report, and to store the one or more report elements in the report data database.

9. A report data database configured to store one or more report elements included in a structured report in one or more database tables.

10. A report data manager configured to provide data storage for report elements included in a qualifying structured report in a predictable set of tables and columns.
